# EUROPÄISCHE PATENTSCHRIFT

(11) **EP 1 432 715 B1**
(45) Veröffentlichungstag und Bekanntmachung des Hinweises auf die Patenterteilung: **19.03.2008**
(21) Anmeldenummer: 02800556.9
(22) Anmeldetag: 05.09.2002
(51) Int. Cl.: C07D 487/04, C07D 495/04, A61K 31/505, A61P 9/08

(54) **PYRIMIDINDERIVATE**
PYRIMIDINE DERIVATIVES
DERIVES DE PYRIMIDINE

(30) Priorität: 04.10.2001 DE 10148883
(43) Veröffentlichungstag der Anmeldung: 30.06.2004
(73) Patentinhaber: Merck Patent GmbH, 64293 Darmstadt (DE)
(72) Erfinder: EGGENWEILER, Hans-Michael, 64287 Darmstadt (DE); BAUMGARTH, Manfred, 64297 Darmstadt (DE); SCHELLING, Pierre, 64367 Mühltal (DE); BEIER, Norbert, 64354 Reinheim (DE); CHRISTADLER, Maria, 63322 Rödermark (DE)
(86) Internationale Anmeldenummer: PCT/EP2002/009935
(87) Internationale Veröffentlichungsnummer: WO 2003/031447

(56) Entgegenhaltungen:
- EP-A- 0 046 677
- EP-A- 0 514 540
- EP-A- 0 728 759
- WO-A-01/18004
- WO-A-01/21620
- WO-A-01/39777
- WO-A-98/17668
- WO-A-99/28325
- WO-A-99/55708
- WO-A-99/62518
- WO-A-02/057267
- US-A- 3 037 980
- US-A- 4 007 187
- CHEMICAL ABSTRACTS, vol. 80, no. 9, 4. März 1974 (1974-03-04) Columbus, Ohio, US; abstract no. 48031, MATSUDA, TAKUMI ET AL: "Furopyrimidine derivatives" XP002224296 -& JP 48 081894 A (HISAMITSU PHARMACEUTICAL CO., INC.) 1. November 1973 (1973-11-01)
- CAMPBELL R M ET AL: "Selective A1-adenosine receptor antagonists identified using yeast saccharomyces cerevisiae functional assays" BIOORGANIC & MEDICINAL CHEMISTRY LETTERS, OXFORD, GB, Bd. 9, Nr. 16, 16. August 1999 (1999-08-16), Seiten 2413-2418, XP004174201 ISSN: 0960-894X
- MEADE E A ET AL: "Anxiolytic activity of analogues of 4-benzylamino-2-methyl-7H-pyrrolo [2,3-d]pyrimidines" EUROPEAN JOURNAL OF MEDICINAL CHEMISTRY, EDITIONS SCIENTIFIQUE ELSEVIER, PARIS, FR, Bd. 33, Nr. 5, 1. Juni 1998 (1998-06-01), Seiten 363-374, XP004127368 ISSN: 0223-5234
- WEST ET AL.: "2-Alkyl(aryl)-and 2,7-dimethyl-4-substituted amino-pyrrolo[2,3-d]pyrimidines" J.ORG.CHEM, Bd. 26, 1961 - 3809, Seite 3812 XP002237089
- CHEMICAL ABSTRACTS, vol. 82, no. 13, 31. März 1975 (1975-03-31) Columbus, Ohio, US; abstract no. 82224, HECHT ET AL.: "Competitive inhibition of beef heart ..." XP002237091 & PROC.NATL.ACAD.SCI.USA, Bd. 71, Nr. 12, 1974, Seite 4670-4674
- CHEMICAL ABSTRACTS, vol. 76, no. 21, 22. Mai 1972 (1972-05-22) Columbus, Ohio, US; abstract no. 126924, MARQUET, JEAN P. ET AL: "New series of purine analogs with antimitotic action. Structure activity relations" XP002237092 & CHIMICA THERAPEUTICA (1971), 6(6), 427-38 ,
- CHEMICAL ABSTRACTS, vol. 63, no. 11, 1965 Columbus, Ohio, US; DANILOV ET AL.: "Excess and obstacle-resistance of the protein synthesis code" Spalte 15130g; XP002237093 & DOKL.AKAD.NAUK SSSR, Bd. 163, Nr. 5, 1963, Seiten 1282-1284,
- LOCKHART ET AL.: "Synthesis of subsituted furo..." J. HETEROCYCLIC CHEM., Bd. 33, Nr. 3, 1996, Seiten 659-661, XP002237090
- MARQUET J-P ET AL: "FURANNES ET PYRROLES DISUBSTITUES EN 2,3. VIII. - NOUVELLE METHODE DE SYNTHESE DES FURO2,3-DPYRIMIDINES SUBSTITUEES EN POSITION 4 ET DE CERTAINS THIENO2,3-DPYRIMIDINES 2,3-DISUBSTITUTED FURANS AND PYRROLES. VIII. NEW SYNTHETIC METHOD FOR 4-SUBSTITUT" BULLETIN DE LA SOCIETE CHIMIQUE DE FRANCE, SOCIETE FRANCAISE DE CHIMIE. PARIS, FR, Nr. 12, 1969, Seiten 4344-4348, XP001095615 ISSN: 0037-8968
- CHEMICAL ABSTRACTS, vol. 111, no. 11, 11. September 1989 (1989-09-11) Columbus, Ohio, US; abstract no. 97174, STADLBAUER: "Synthesis of benzofuro..." XP002237094 & MONATSH.CHEM., Bd. 120, Nr. 1, 1987, Seiten 77-80,
- CHEMICAL ABSTRACTS, vol. 123, no. 9, 28. August 1995 (1995-08-28) Columbus, Ohio, US; abstract no. 112617, RENAULT ET AL.: "Synthesis and antiviral evaluation..." XP002237095 & HETEROCYCLES, Bd. 41, Nr. 5, 1995, Seiten 937-945,
- CHEMICAL ABSTRACTS, vol. 83, no. 9, 1. September 1975 (1975-09-01) Columbus, Ohio, US; abstract no. 79191, WAMHOFF ET AL.: "Hetorcyclic beta-enamino esters..." XP002237096 & CHEM.BER., Bd. 108, Nr. 6, 1975, Seiten 2107-2114,

## Beschreibung

Die Erfindung betrifft einzelne Verbindungen gemäss Anspruch 1 umfasst von der Formel I 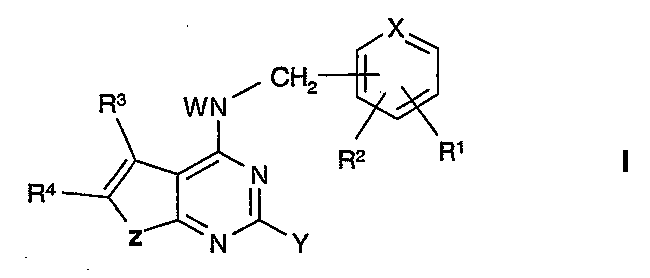 worin
- R¹, R²: jeweils unabhängig voneinander H, A, OH, OA oder Hal,
- R¹ und R²: zusammen auch Alkylen mit 3-5 C-Atomen, -O-CH₂-CH₂-, -CH₂-O-CH₂-, -O-CH₂-O- oder -O-CH₂-CH₂-O-,
- R³, R⁴: jeweils unabhängig voneinander H, A oder Hal,
- R³ und R⁴: zusammen auch Alkylen oder Alkyliden mit 3-5 C-Atomen, oder 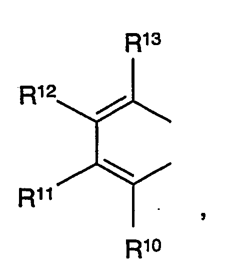
- W: H oder Alkyl mit 1, 2, 3 oder 4 C-Atomen,
- X: CH oder N,
- Y: (CH₂)_{q}-R⁷ oder unsubstituiertes oder einfach durch (CH₂)ₙR²⁰ substituiertes R⁵, R⁶ oder R⁹,
- Z: O, NH oder NA',
- A': Alkyl mit 1-6 C-Atomen, -CHAr oder -CHAr-A",
- A": Alkyl mit 1-6 C-Atomen,
- R⁵: lineares oder verzweigtes Alkyl mit 1-10 C-Atomen, worin eine oder zwei CH₂-Gruppen durch -CH=CH-Gruppen, -C≡C-Gruppen, -O-, CO, -S-, -SO-, SO₂, -NH- oder -NA- ersetzt sein können,
- R⁶: Cycloalkyl oder Cycloalkylalkylen mit 5-12 C-Atomen,
- R⁷: einen unsubstituierten oder ein-, zwei- oder dreifach durch R²⁰, A, Hal oder CF₃ substituierten, gesättigten oder ungesättigten 5-7-gliedrigen Heterocyclus mit 1-4 N-, O- und/oder S-Atomen,
- R⁹: Ar oder (CH₂)ₖ-Ar,
- R¹⁰, R¹¹, R¹², R¹³: jeweils unabhängig voneinander H, A, Hal, OA, OH, NH₂, NHA, NA₂ oder R²⁰,
- R²⁰: -COOH, -COOA, -CONH₂, -CONHA, -CONA₂, -CN, Tetrazol-5-yl, -S(O)ₘA, -S(O)ₘNH₂ oder -S(O)ₘOH, 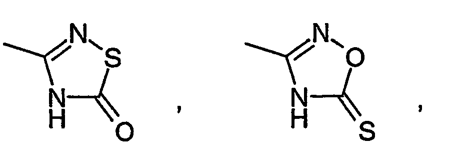 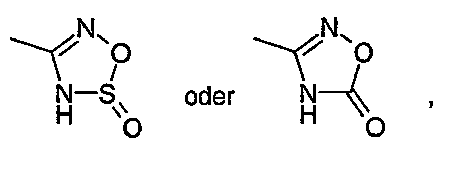
- A: Alkyl oder Alkenyl mit 1 bis 6 C-Atomen, worin 1-7 H-Atome durch F ersetzt sein können,
- Ar: einen unsubstituierten oder ein-, zwei- oder dreifach durch Hal, A, OA, OH, SO₂NH₂, SO₂NHA, SO₂NA₂ oder CN substituierten Phenylrest,
- Hal: F, Cl, Br oder 1,
- k, q: jeweils unabhängig voneinander 0, 1, 2, 3 oder 4,
- m: 1 oder 2
und
- n: 0, 1, 2 oder 3
bedeuten,
sowie ihre pharmazeutisch verwendbaren Derivate, Solvate und Stereoisomere, einschließlich deren Mischungen in allen Verhältnissen.

Pyrimidinderivate sind beispielsweise aus der EP 0920431, EP 0934321, WO 99/28325, WO 99/55708, WO 00/78767 und WO 01/21620 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die beanspruchte Verbindungen der Formel I und ihre Salze und/oder Solvate bei guter Verträglichkeit sehr wertvolle pharmakologische Eigenschaften besitzen.

Insbesondere zeigen sie eine spezifische Inhibierung der cGMP-Phosphodiesterase (PDE V).

Im Vergleich zu den Verbindungen aus dem Stand der Technik, weisen die erfindungsgemäßen Verbindungen günstigere physikalisch-chemische Eigenschaften auf. So sind sie besser löslich und werden z.B. bei oraler Gabe besser resorbiert.

Chinazoline mit cGMP-Phosphodiesterase hemmender Aktivität sind z.B. in J. Med. Chem. 36, 3765 (1993) und ibid. 37, 2106 (1994) beschrieben.

Die biologische Aktivität der Verbindungen der Formel 1 kann nach Methoden bestimmt werden, wie sie z.B in der WO 93/06104 beschrieben sind.

Die Affinität der erfindungsgemäßen Verbindungen für cGMP- und cAMP-Phosphodiesterase wird durch die Ermittlung ihrer IC₅₀-Werte (Konzentration des Inhibitors, die benötigt wird, um eine 50 %ige Inhibierung der Enzymaktivität zu erreichen) bestimmt.
Zur Durchführung der Bestimmungen können nach bekannten Methoden isolierte Enzyme verwendet werden (z.B. W.J. Thompson et al., Biochem. 1971, 10, 311). Zur Durchführung der Versuche kann eine modifizierte "batch"-Methode von W.J: Thompson und M.M. Appleman (Biochem. 1979, 18, 5228) angewendet werden.

Die in US 6,043,252 beschriebenen Carbolinderivate sind cGMPspezifische PDE (PDE V) - Hemmer und eignen sich zur Behandlung einer Vielzahl von Krankheiten.

Die erfindungsgemäßen Verbindungen eignen sich daher zur Behandlung von Erkrankungen des Herz-Kreislaufsystems, insbesondere der Herzinsuffizienz und zur Behandlung und/oder Therapie von Potenzstörungen (erektile Dysfunktion).

Die erfindungsgemäßen Verbindungen eignen sich weiterhin zur Behandlung von Angina, Bluthochdruck, pulmonarem Hochdruck, congestivem Herzversagen, Herzinfarkt, chronischer obstruktiver pulmonaler Krankheit (COPD), Cor pulmonale, Rechtsherzinsuffizienz, Atherosklerose, Bedingungen verminderter Durchgängigkeit der Herzgefäße, peripheren vaskulären Krankheiten, Schlaganfall, Bronchitis, allergischem Asthma, chronischem Asthma, allergischer Rhinitis, Glaucom, Irritable Bowel Syndrome, Tumoren, Niereninsuffizienz, Leberzirrhose, zur Behandlung weiblicher Sexualstörungen, Entzündungen, Osteoporose, weiterhin zur Behandlung von maligner Hypertonie, Phäochromazytom (katecholaminproduzierender Tumor der Nebennierenrinde), bei peripheren Gefäß(-verschluß)-erkrankungen, Gefäßerkrankungen, Thrombozytopenie, Ulcus pepticum (gutartiges Darmgeschwür), Darmbewegungsstörungen, perkutaner transluminaler Koronarangioplastie, Karotis Angioplastie, postoperativer Stenose des Koronarbypasses, Vorwehen sowie benigner Prostata-Hyperplasie.

Die Verwendung von substituierten Pyrazolopyrimidinonen zur Behandlung von Impotenz ist z.B. in der WO 94/28902 beschrieben.

Die Verbindungen sind wirksam als Inhibitoren der Phenylephrin-induzierten Kontraktionen in Corpus cavemosum-Präparationen von Hasen.
Diese biologische Wirkung kann z.B. nach der Methode nachgewiesen werden, die von F. Holmquist et al. in J. Urol., 150, 1310-1315 (1993) beschrieben wird.
Die Inhibierung der Kontraktion, zeigt die Wirksamkeit der erfindungsgemäßen Verbindungen zur Therapie und/oder Behandlung von Potenzstörungen.

Pharmazeutische Formulierungen bestehend aus anderen Phosphodiesterase V (PDE V)-Hemmern zusammen mit einem zweiten Wirkstoff sind in der WO 00/15639 beschrieben.
Andere Kombinationen kennt man aus der WO 00/15228.

Pharmazeutische Formulierungen bestehend aus anderen Phosphodiesterase V (PDE V)-Hemmern zusammen mit einem Prostaglandin oder Prostaglandinderivat sind in der WO 00/15639 und WO 0015228 beschrieben.

Die Verwendung von (anderen) Phosphodiesterase IV oder V Hemmern in Kombination mit einem Prostaglandin oder Prostaglandinderivat zur lokalen Behandlung von erektiler Dysfunktion ist in der WO 9921558 beschrieben.

R.T. Schermuly et al. beschreiben im American Journal of Respiratory and Critical Care Medicine, 160, 1500-6 (1999), die therapeutische Möglichkeit Prostaglandin I₂ (PGI₂) in Aerosolform mit systemischen PDE Inhibitoren, vorzugsweise dual-selektiven PDE III/IV Inhibitoren, in niedriger Dosierung bei akutem und chronischen pulmonalem Hochdruck zu verwenden.

In Pneumologie (54, Suppl. 1, S42, 2000) wird von R. Schermuly et al. der Einfluß der PDE V-Inhibierung auf die durch Prostacyclin induzierte Vasorelaxation bei experimenteller pulmonaler Hypertonie beschrieben.

Pharmazeutische Formulierungen bestehend aus anderen Phosphodiesterase V (PDE V)-Hemmern zusammen mit Calcium-Antagonisten (= Calciumkanalblocker) sind in der WO 00/15639 beschrieben.

Kombinationen von PDE V - Hemmern mit Endothelin-Rezeptor-Antagonisten sind z.B in der WO 99/64004 beschrieben.

Pharmazeutische Formulierungen bestehend aus anderen Phosphodiesterase V (PDE V)-Hemmern zusammen mit einem Nitrat sind in der WO 00/15228 beschrieben.
Die bekannte Kontraindikation der Gabe von Nitraten bei gleichzeitiger Einnahme von PDE V - Hemmern bei der Indikation erektile Dysfunktion ist z.B. in der WO 00/10542 beschrieben. Gleichzeitig wird dort jedoch offenbart, daß Nitrate als antianginöse Mittel verabreicht werden können, obwohl gleichzeitig Phosphodiesterase V - Hemmer zur Behandlung erektiler Dysfunktion eingesetzt werden.
Weiter werden dort pharmazeutische Zubereitungen beschrieben, die sowohl ein Nitrat als auch einen Phosphodiesterasehemmer enthalten, zur Anwendung in der Therapie erektiler Dysfunktion und/oder in der Therapie von Herz- /Kreislauferkrankungen bei gleichzeitigem Vorliegen der jeweils anderen Indikation.

Die Verbindungen der Formel I können als Arzneimittelwirkstoffe in der Human- und Veterinärmedizin eingesetzt werden. Ferner können sie als Zwischenprodukte zur Herstellung weiterer Arzneimittelwirkstoffe eingesetzt werden.

Gegenstand der Erfindung sind dementsprechend die Verbindungen der Formel I gemäß Anspruch 1 sowie ein Verfahren zur Herstellung
von Verbindungen der Formel 1 nach Anspruch 1 sowie deren Salzen,
dadurch gekennzeichnet, daß man
a) eine Verbindung der Formel II 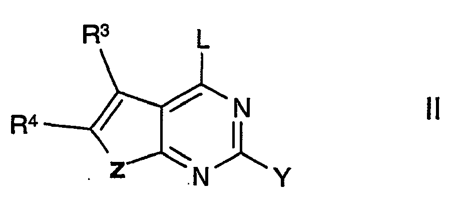 worin
   Y, Z, R¹ und R² die in Anspruch 1 angegebenen Bedeutungen haben,
   und L Cl, Br, OH, SCH₃ oder eine reaktionsfähige veresterte OH-Gruppe bedeutet,
   mit einer Verbindung der Formel III 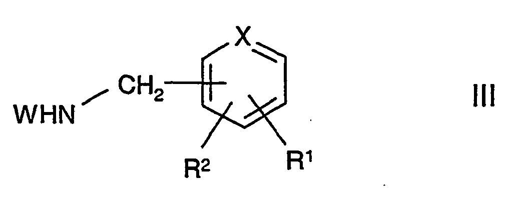 worin
   X, W, R¹ und R² die in Anspruch 1 angegebenen Bedeutungen haben, umsetzt,
   oder
b) in einer Verbindung der Formel I einen Rest X in einen anderen Rest X umwandelt, indem man z.B. eine Estergruppe zu einer COOH-Gruppe hydrolysiert oder eine COOH-Gruppe in ein Amid oder in eine Cyangruppe umwandelt
und/oder daß man eine Verbindung der Formel I in eines ihrer Salze überführt.

Gegenstand der Erfindung sind auch die optisch aktiven Formen (Stereoisomeren), die Enantiomeren, die Racemate, die Diastereomeren sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Unter pharmazeutisch verwendbaren Derivaten versteht man z.B. die Salze der erfindungsgemäßen Verbindungen als auch sogenannte Prodrug-Verbindungen.
Unter Prodrug-Derivaten versteht man mit z. B. Alkyl- oder Acylgruppen, Zuckern oder Oligopeptiden abgewandelte Verbindungen der Formel I, die im Organismus rasch zu den wirksamen erfindungsgemäßen Verbindungen gespalten werden.
Hierzu gehören auch bioabbaubare Polymerderivate der erfindungsgemäßen Verbindungen, wie dies z. B. in Int. J. Pharm. 115, 61-67 (1995) beschrieben ist.

Gegenstand der Erfindung sind auch Mischungen der erfindungsgemäßen Verbindungen, z.B. Gemische zweier Diastereomerer z.B. im Verhältnis 1:1, 1:2, 1:3, 1:4, 1:5, 1:10, 1:100 oder 1:1000.
Besonders bevorzugt handelt es sich dabei um Mischungen stereoisomerer Verbindungen.

Vor- und nachstehend haben die Reste R¹, R², R³, R⁴, W, X, Y, Z und L die bei den Formeln I, II und III angegebenen Bedeutungen, sofern nicht ausdrücklich etwas anderes angegeben ist.
A bedeutet Alkyl mit 1-6 C-Atomen.
In den vorstehenden Formeln ist Alkyl vorzugsweise unverzweigt und hat 1, 2, 3, 4, 5 oder 6 C-Atome und bedeutet vorzugsweise Methyl, Ethyl oder Propyl, weiterhin bevorzugt Isopropyl, Butyl, Isobutyl, sek.-Butyl oder tert.-Butyl, aber auch n-Pentyl, Neopentyl, Isopentyl oder Hexyl.
A bedeutet weiterhin Alkenyl mit 2-6 C-Atomen, z.B. Vinyl oder Propenyl.
A bedeutet ferner einen halogenierten Alkylrest, wie z.B. Trifluormethyl.

A' bedeutet vorzugsweise Alkyl mit 1, 2, 3, 4, 5 oder 6 C-Atomen, -CHAr oder -CHAr-A", wobei Ar vorzugsweise Phenyl bedeutet.
A" bedeutet vorzugsweise Alkyl mit 1, 2, 3, 4, 5 oder 6 C-Atomen.

Hal bedeutet vorzugsweise F, Cl oder Br, aber auch I.

X bedeutet vorzugsweise CH, ferner N.

Die Reste R¹ und R² können gleich oder verschieden sein und stehen vorzugsweise in der 3- oder 4-Position des Phenylrings. Sie bedeuten beispielsweise jeweils unabhängig voneinander H, OH, Alkyl, F, Cl, Br oder I oder zusammen Ethylenoxy, Methylendioxy oder Ethylendioxy. Bevorzugt stehen sie auch jeweils für Alkoxy, wie z.B. für Methoxy, Ethoxy oder Propoxy.
R¹ bedeutet insbesondere 3-H, 3-Cl oder 3-Methoxy.

R² bedeutet insbesondere 4-H, 4-Chlor oder 4-Methoxy.
R¹ und R² zusammen bedeuten insbesondere -OCH₂O-.

Der Heterocyclus in R⁷ bedeutet vorzugsweise 2- oder 3-Furyl, 2- oder 3-Thienyl, 1-, 2- oder 3-Pyrrolyl, 1-, 2-, 4- oder 5-lmidazolyl, 2-Methyl-1-imidazol-1-yl, 1-, 3-, 4- oder 5-Pyrazolyl, 2-, 4- oder 5-Oxazolyl, 3-, 4- oder 5-lsoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, - 4- oder -5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl oder Pyrazinyl.

R³ und R⁴ bedeuten zusammen vorzugsweise -(CH₂)₃-, -(CH₂)₄- oder -CH=CH-CH=CH-.

R⁵ bedeutet einen linearen oder verzweigten Alkylrest mit 1-10 C-Atomen, wobei der Alkylrest vorzugsweise z.B. Methyl, Ethyl, Propyl, Isopropyl, Butyl, Isobutyl, sek.-Butyl, Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, lineares oder verzweigtes Heptyl, Octyl, Nonyl oder Decyl bedeutet.
R⁵ bedeutet ferner z.B. But-2-en-yl oder Hex-3-en-yl.
Ganz besonders bevorzugt ist Methyl, Ethyl, Propyl oder Butyl, wobei vorzugsweise eine CH₂-Gruppe durch O ersetzt ist.

R⁶ bedeutet Cycloalkylalkylen mit 5-12 C-Atomen, vorzugsweise z.B. Cyclopentylmethylen, Cyclohexylmethylen, Cyclohexylethylen, Cyclohexylpropylen oder Cyclohexylbutylen.

R⁶ bedeutet auch Cycloalkyl mit vorzugsweise mit 5-7 C-Atomen. Cycloalkyl bedeutet z.B. Cyclopentyl, Cyclohexyl oder Cycloheptyl.

R⁷ bedeutet z.B. 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder-5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7- Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, weiter bevorzugt 1,3-Benzodioxol-5-yl, 1,4-Benzodioxan-6-yl, 2,1,3-Benzothiadiazol-4- oder-5-yl oder 2,1,3-Benzoxadiazol-5-yl.
Die heterocyclischen Reste können auch teilweise oder vollständig hydriert sein.
R⁷ kann also z. B. auch bedeuten 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4- oder-5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3- oder -4-pyridyl, 1,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder-4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-,-2-,-3-, -4-, -5-, -6-, -7- oder -8-isochinolyl, 2-, 3-, 5-, 6-, 7- oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydrobenzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydrobenzofuranyl oder 2,3-Dihydro-2-oxo-furanyl.
R⁷ bedeutet ganz besonders bevorzugt Piperidyl, Pyrrolidinyl oder piperazinyl.

R¹⁰, R¹¹, R¹², R¹³ bedeuten ganz besonders bevorzugt H.

Ar bedeutet z.B. unsubstituiertes Phenyl, Naphthyl oder Biphenyl, weiterhin vorzugsweise z.B. durch A, Fluor, Chlor, Brom, Iod, Hydroxy, Methoxy, Ethoxy, Propoxy, Butoxy, Pentyloxy, Hexyloxy, Nitrö, Cyan, Formyl, Acetyl, Propionyl, Trifluormethyl, Amino, Methylamino, Ethylamino, Dimethylamino, Diethylamino, Benzyloxy, Sulfonamido, Methylsulfonamido, Ethylsulfonamido, Propylsulfonamido, Butylsulfonamido, Dimethylsulfonamido, Phenylsulfonamido, Carboxy, Methoxycarbonyl, Ethoxycarbonyl, Aminocarbonyl mono-, di- oder trisubstituiertes Phenyl, Naphthyl oder Biphenyl. Ar bedeutet ganz besonders bevorzugt Phenyl.

Für die gesamte Formel I gilt, daß sämtliche Reste, die mehrfach auftreten, gleich oder verschieden sein können, d.h. unabhängig voneinander sind.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart), beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

In den Verbindungen der Formeln II oder III haben R¹, R² und X die angegebenen Bedeutungen.

Falls L eine reaktionsfähige veresterte OH-Gruppe bedeutet, so ist diese vorzugsweise Alkylsulfonyloxy mit 1-6 C-Atomen (bevorzugt Methylsulfonyloxy) oder Arylsulfonyloxy mit 6-10 C-Atomen (bevorzugt Phenyl- oder p-Tolylsulfonyloxy, ferner auch 2-Naphthalinsulfonyloxy).

Die Verbindungen der Formel 1 können vorzugsweise erhalten werden, indem man Verbindungen der Formel II mit Verbindungen der Formel III umsetzt.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.
Andererseits ist es möglich, die Reaktion stufenweise durchzuführen.

Die Ausgangsverbindungen der Formel II und III sind in der Regel bekannt. Sind sie nicht bekannt, so können sie nach an sich bekannten Methoden hergestellt werden.

Die Verbindungen der Formel II werden in der Regel aus den entsprechenden 2-Amino-indol- oder 2-Aminofuranderivaten hergestellt.

Die Herstellung von Verbindungen der Formel II 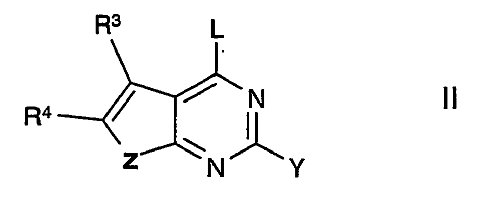 worin
- Z: NH oder NHA',
- L: Cl,
- Y: unsubstituiertes oder einfach durch (CH₂)ₙR²⁰ substituiertes R⁵, R⁶ oder R⁹,
- R³, R⁴: jeweils unabhängig voneinander H, A oder Hal,
- R³ und R⁴: zusammen auch Alkylen oder Alkyliden mit 3-5 C-Atomen, bedeuten,
und n, R²⁰, R⁵, R⁶ und R⁹ die in Anspruch 1 entsprechenden Bedeutungen haben,
erfolgt z.B. analog nachstehendem Schema 1

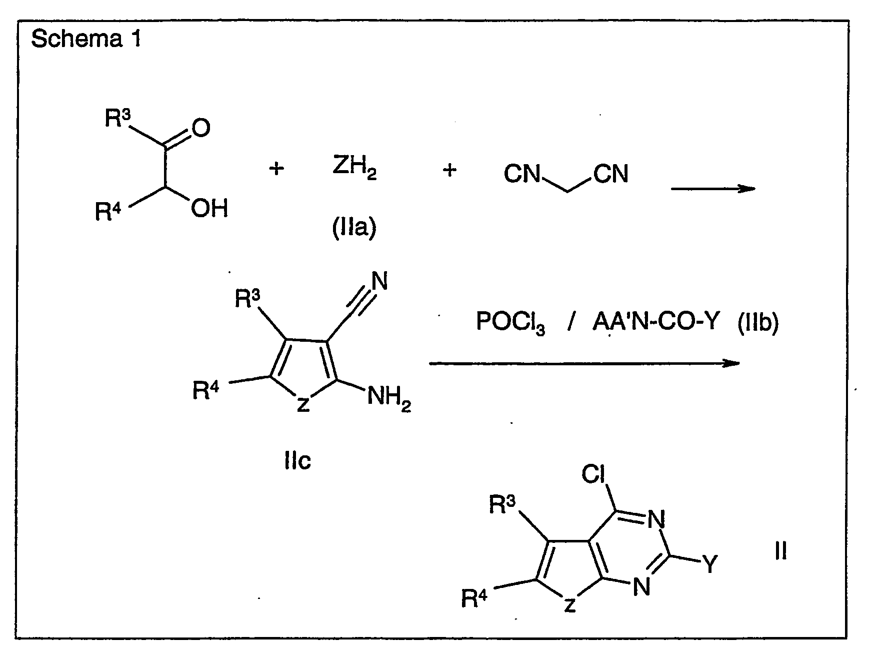

In allen Formeln des Schema 1 bedeuten
- R³, R⁴: jeweils unabhängig voneinander H, A oder Hal,
- R³ und R⁴: zusammen auch Alkylen oder Alkyliden mit 3-5 C-Atomen.
In den Verbindungen der Formel IIa bedeutet Z NH oder NA', wobei A' die in Anspruch 1 entsprechende Bedeutung hat.
In den Verbindungen der Formel IIb bedeutet
- A,A': jeweils unabhängig voneinander H oder Alkyl mit 1, 2, 3 oder 4 C-Atomen, und
- Y: unsubstituiertes oder einfach durch (CH₂)ₙR²⁰ substituiertes R⁹, R⁶ oder R⁹,
und wobei n, R²⁰, R⁵, R⁶ und R⁹ die in Anspruch 1 entsprechenden Bedeutungen haben.
In den Verbindungen der Formel IIc bedeutet Z NH oder NA', wobei A' die in Anspruch 1 entsprechende Bedeutung hat.

Die Herstellung von Verbindungen der Formel II 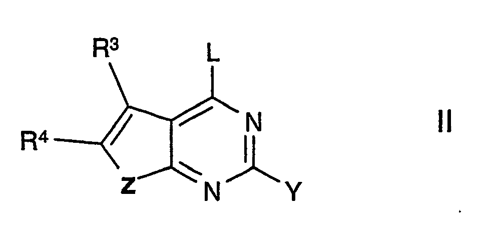 worin
- Z: O,
- L: Cl,
- Y: unsubstituiertes oder einfach durch (CH₂)ₙR²⁰ substituiertes R⁵, R⁶ oder R⁹,
- R³, R⁴: jeweils unabhängig voneinander H, A oder Hal,
- R³ und R⁴: zusammen auch Alkylen oder Alkyliden mit 3-5 C-Atomen bedeuten,
und n, R²⁰, R⁵, R⁶ und R⁹ die in Anspruch 1 entsprechenden Bedeutungen haben,
erfolgt z.B. analog nachstehendem Schema 2

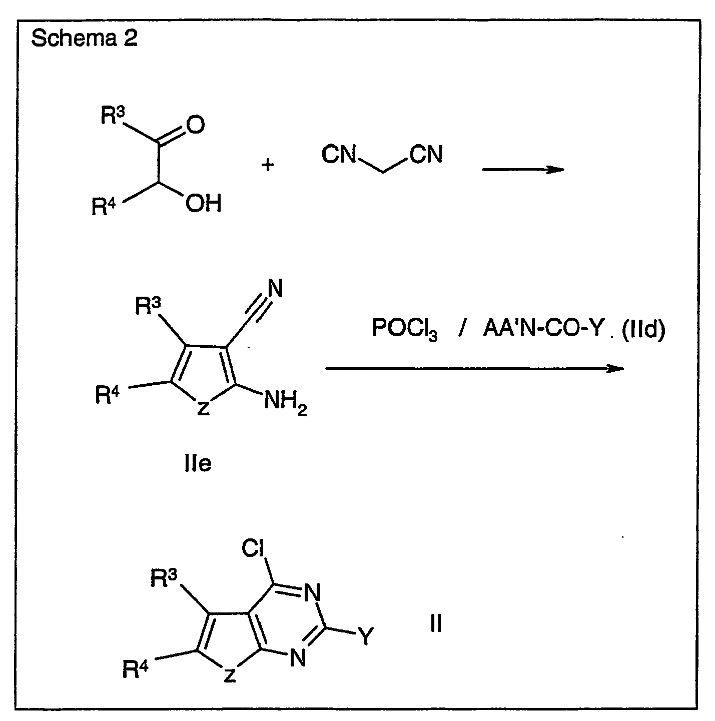

In allen Formeln des Schema 2 bedeuten
- R³, R⁴: jeweils unabhängig voneinander H, A oder Hal,
- R³ und R⁴: zusammen auch Alkylen oder Alkyliden mit 3-5 C-Atomen.

In den Verbindungen der Formel IId bedeutet
- A,A': jeweils unabhängig voneinander H oder Alkyl mit 1, 2, 3 oder 4 C-Atomen, und
- Y: unsubstituiertes oder einfach durch (CH₂)ₙR²⁰ substituiertes R⁵, R⁶ oder R⁹,
und wobei n, R²⁰, R⁵, R⁶ und R⁹ die in Anspruch 1 entsprechenden Bedeutungen haben.

In den Verbindungen der Formel IIe bedeutet Z O (Sauerstoff).

Die Herstellung von Verbindungen der Formel II 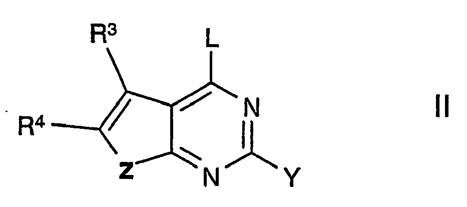 worin
- Z: O,
- L: Cl,
- Y: (CH₂)_{q}-R⁷,
- R³, R⁴: jeweils unabhängig voneinander H, A oder Hal,
- R³ und R⁴: zusammen auch Alkylen oder Alkyliden mit 3-5 C-Atomen bedeuten,
- q, R⁷: die in Anspruch 1 entsprechenden Bedeutungen haben,
und wobei R⁷ mindestens ein N-Atom enthält,
erfolgt z.B. analog nachstehendem Schema 3

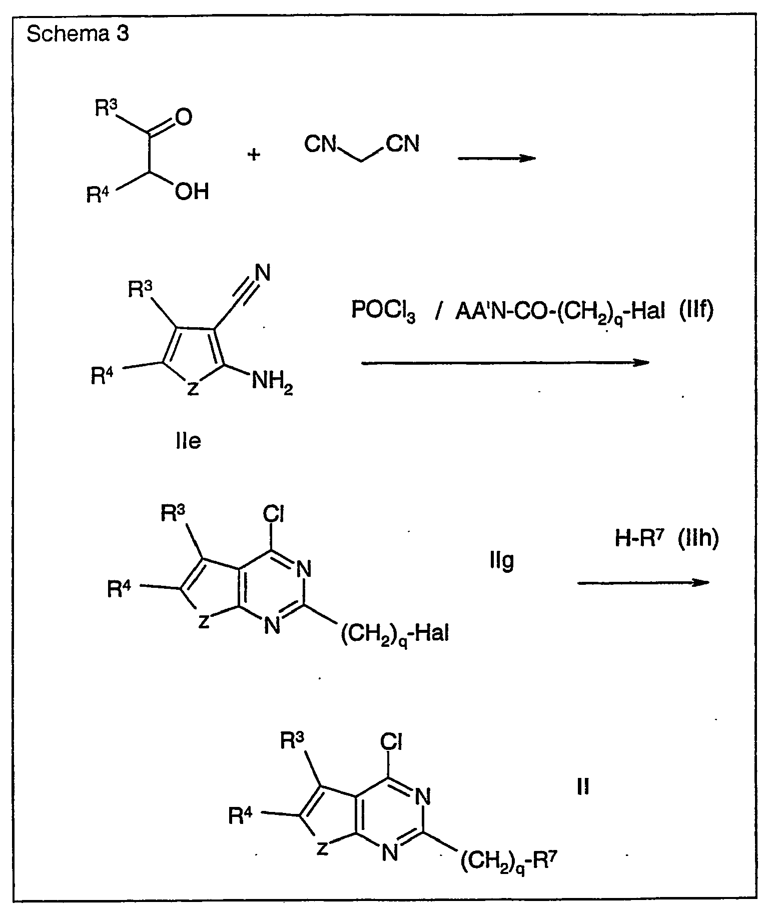

In allen Formeln des Schema 3 bedeuten
- R³, R⁴: jeweils unabhängig voneinander H, A oder Hal,
- R³ und R⁴: zusammen auch Alkylen oder Alkyliden mit 3-5 C-Atomen.
In den Verbindungen der Formel IIf bedeutet
- A,A': jeweils unabhängig voneinander H oder Alkyl mit 1, 2, 3 oder 4 C-Atomen, und
- Hal: Cl oder Br.
In den Verbindungen der Formel IIg bedeutet
- Z: O,
- q: 0, 1, 2, 3 oder 4,

- Hal: Br oder Cl,

In den Verbindungen der Formel IIh hat R⁷ die in Anspruch 1 angegebene Bedeutung, wobei der Heterocyclus mindestens einen substituierbaren Stickstoff enthält.

Die Herstellung von Verbindungen der Formel II 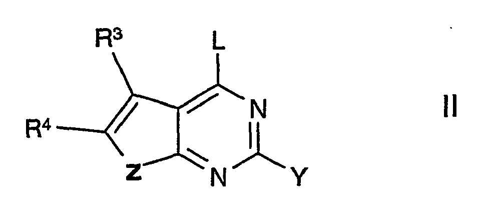 worin
- Z: NH,
- L: Cl,
- Y: unsubstituiertes oder einfach durch (CH₂)ₙR²⁰ substituiertes R⁵, R⁶ oder R⁹,
- R³ und R⁴: zusammen 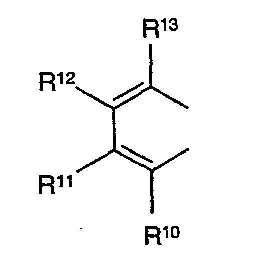
bedeuten,
n, R²⁰, R⁵, R⁶ und R⁹ die in Anspruch 1 entsprechenden Bedeutungen haben, erfolgt z.B. analog nachstehendem Schema 4

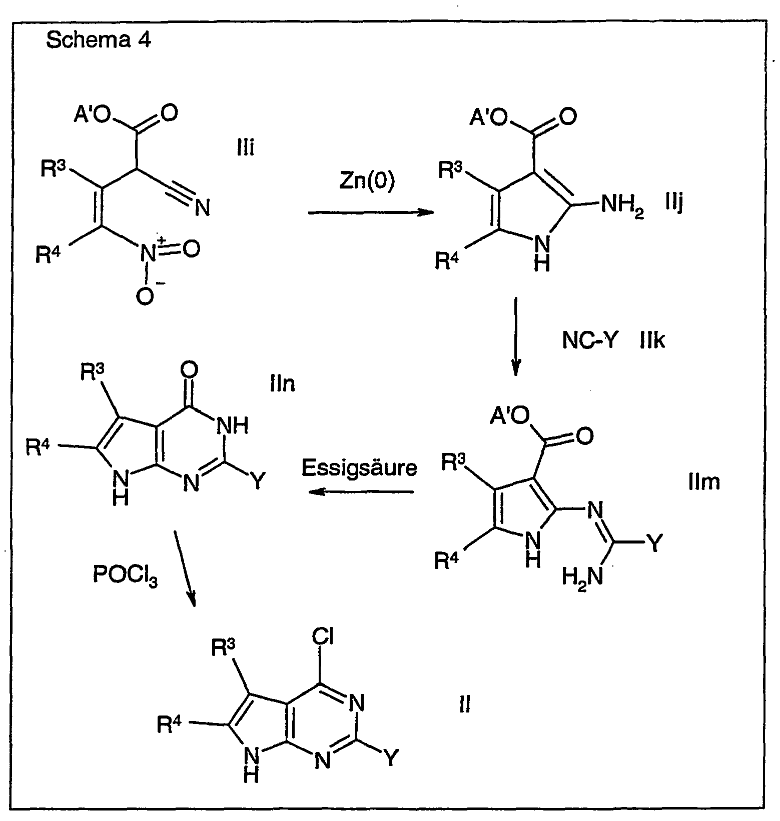

In allen Formeln des Schema 4 bedeuten
- R³ und R⁴: zusammen 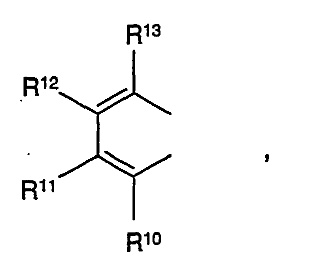
wobei R¹⁰, R¹¹, R¹², R¹³ die in Anspruch 1 entsprechenden Bedeutungen haben.

Die Herstellung der Verbindungen der Formel IIi erfolgt z.B. analog I.T. Fordes, C.N. Johnson, M. Thompson, J. Chem. Soc. Perkin Trans. 1, 2, 275-282 (1992).
In den Verbindungen der Formel III bedeutet A' Alkyl mit 1, 2, 3 oder 4 C-Atomen.
In den Verbindungen der Formel IIj bedeutet A' Alkyl mit 1, 2, 3 oder 4 C-Atomen.
In den Verbindungen der Formel IIk bedeutet Y unsubstituiertes oder einfach durch (CH₂)ₙR²⁰ substituiertes R⁵, R⁶ oder R⁹, wobei n, R²⁰, R⁵, R⁶ und R⁹ die in Anspruch 1 entsprechenden Bedeutungen haben.
In den Verbindungen der Formel IIm bedeutet A' Alkyl mit 1, 2, 3 oder 4 C-Atomen und Y bedeutet unsubstituiertes oder einfach durch (CH₂)ₙR²⁰ substituiertes R⁵, R⁶ oder R⁹, wobei n, R²⁰, R⁵, R⁶ und R⁹ die in Anspruch 1 entsprechenden Bedeutungen haben.
In den Verbindungen der Formel IIn bedeutet Y unsubstituiertes oder einfach durch (CH₂)ₙR²⁰ substituiertes R⁹, R⁶ oder R⁹, wobei n, R²⁰, R⁵, R⁶ und R⁹ die in Anspruch entsprechenden Bedeutungen haben.

Die Herstellung von Verbindungen der Formel II 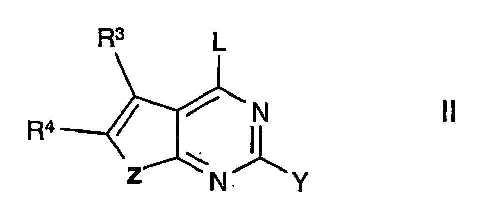 worin
- Z: NA',
- L: Cl,
- Y: unsubstituiertes oder einfach durch (CH₂)ₙR²⁰ substituiertes R⁵, R⁶ oder R⁹,
- R³ und R⁴: zusammen 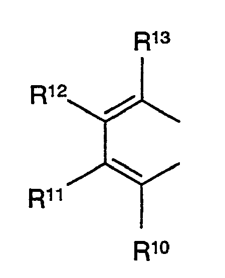
bedeuten,
A', n, R²⁰, R⁵, R⁶ und R⁹ die in Anspruch 1 entsprechenden Bedeutungen haben,
erfolgt z.B. analog nachstehendem Schema 5

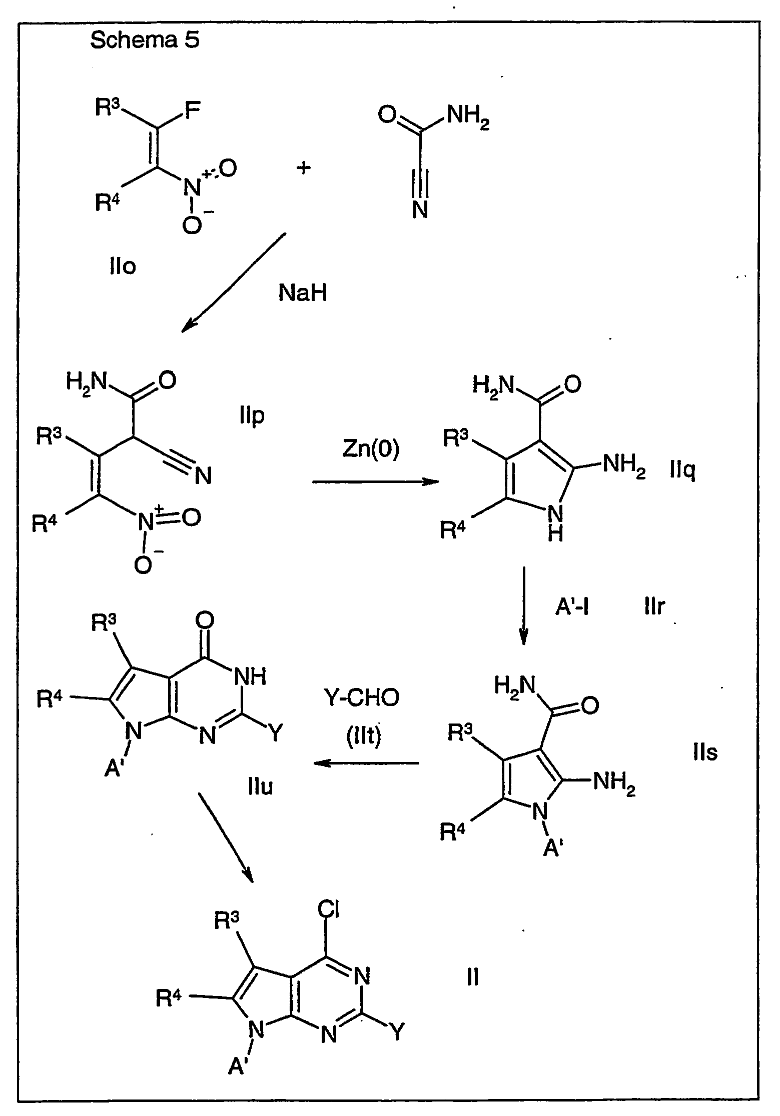

In allen Formeln des Schema 5 bedeuten
- R³ und R⁴: zusammen 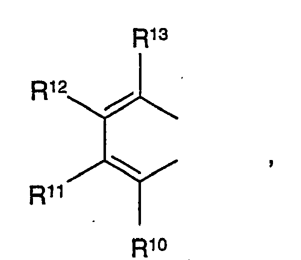
wobei R¹⁰, R¹¹, R¹², R¹³ die in Anspruch 1 entsprechenden Bedeutungen haben.
In den Verbindungen der Formeln IIs und IIu hat A' die in Anspruch 1 entsprechende Bedeutung.
In Verbindung IIt bedeutet Y unsubstituiertes oder einfach durch (CH₂)ₙR²⁰ substituiertes R⁵, R⁶ oder R⁹, wobei n, R²⁰, R⁹, R⁶ und R⁹ die in Anspruch 1 entsprechenden Bedeutungen haben.

Im einzelnen erfolgt die Umsetzung der Verbindungen der Formel II mit den Verbindungen der Formel III in Gegenwart oder Abwesenheit eines inerten Lösungsmittels bei Temperaturen zwischen etwa -20 und etwa 150°, vorzugsweise zwischen 20 und 100°.

Der Zusatz eines säurebindenden Mittels, beispielsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums oder Calciums, oder der Zusatz einer organischen Base wie Triethylamin, Dimethylamin, Pyridin oder Chinolin oder eines Überschusses der Aminkomponente kann günstig sein.

Als inerte Lösungsmittel eignen sich z.B. Kohlenwasserstoffe wie Hexan, Petrolether, Benzol, Toluol oder Xylol; chlorierte Kohlenwassertoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid, N-Methylpyrrolidon oder Dimethylformamid (DMF); Nitrile wie Acetonitril; Sulfoxide wie Dimethylsulfoxid (DMSO); Nitroverbindungen wie Nitromethan oder Nitrobenzol; Ester wie Ethylacetat oder Gemische der genannten Lösungsmittel.

Es ist ferner möglich, in einer Verbindung der Formel I einen Rest X in einen anderen Rest X umzuwandeln, z.B. indem man einen Ester oder eine Cyangruppe zu einer COOH-Gruppe hydrolysiert.
Estergruppen können z.B. mit NaOH oder KOH in Wasser, Wasser-THF oder Wasser-Dioxan bei Temperaturen zwischen 0 und 100° verseift werden.
Carbonsäuren können z.B. mit Thionylchlorid in die entsprechenden Carbonsäurechloride und diese in Carbonsäureamide umgewandelt werden. Durch Wasserabspaltung in bekannter Weise erhält man aus diesen Carbonitrile.

Eine Säure der Formel I kann mit einer Base in das zugehörige Säureadditionssalz übergeführt werden, beispielsweise durch Umsetzung äquivalenter Mengen der Säure und der Base in einem inerten Lösungsmittel wie Ethanol und anschließendes Eindampfen. Für diese Umsetzung kommen insbesondere Basen in Frage, die physiologisch unbedenkliche Salze liefern.
So kann die Säure der Formel I mit einer Base (z.B. Natrium- oder Kaliumhydroxid oder -carbonat) in das entsprechende Metall-, insbesondere Alkalimetall- oder Erdalkalimetall-, oder in das entsprechende Ammoniumsalz umgewandelt werden.
Für diese Umsetzung kommen insbesondere auch organische Basen in Frage, die physiologisch unbedenkliche Salze liefern, wie z.B. Ethanolamin.

Andererseits kann eine Base der Formel I mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Erfindungsgemäße Verbindungen der Formel I gemäss Anspruch 1 können aufgrund ihrer Molekülstruktur chiral sein und können dementsprechend in verschiedenen enantiomeren Formen auftreten. Sie können daher in racemischer oder in optisch aktiver Form vorliegen.

Da sich die pharmazeutische Wirksamkeit der Racemate bzw. der Stereoisomeren der erfindungsgemäßen Verbindungen unterscheiden kann, kann es wünschenswert sein, die Enantiomere zu verwenden. In diesen Fällen kann das Endprodukt oder aber bereits die Zwischenprodukte in enantiomere Verbindungen, durch dem Fachmann bekannte chemische oder physikalische Maßnahmen, aufgetrennt oder bereits als solche bei der Synthese eingesetzt werden.

Im Falle racemischer Amine werden aus dem Gemisch durch Umsetzung mit einem optisch aktiven Trennmittel Diastereomere gebildet. Als Trennmittel eignen sich z.B. optisch aktiven Säuren, wie die R- und S-Formen von Weinsäure, Diacetylweinsäure, Dibenzoylweinsäure, Mandelsäure, Äpfelsäure, Milchsäure, geeignet N-geschützte Aminosäuren (z.B. N-Benzoylprolin oder N-Benzolsulfonylprolin) oder die verschiedenen optisch aktiven Camphersulfonsäuren. Vorteilhaft ist auch eine chromatographische Enantiomerentrennung mit Hilfe eines optisch aktiven Trennmittels (z.B. Dinitrobenzoylphenylglycin, Cellulosetriacetat oder andere Derivate von Kohlenhydraten oder auf Kieselgel fixierte chiral derivatisierte Methacrylatpolymere). Als Laufmittel eignen sich hierfür wäßrige oder alkoholische Lösungsmittelgemische wie z.B. Hexan/Isopropanol/ Acetonitril z.B. im Verhältnis 82:15:3.

Gegenstand der Erfindung ist ferner die Verwendung der Verbindungen der Formel I gemäß Anspruch 1 und/oder ihrer physiologisch unbedenklichen Salze zur Herstellung eines Arzneimittels (pharmazeutische Zubereitung), insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner Arzneimittel, enthaltend mindestens eine Verbindung der Formel gemäß Anspruch 1 und/oder ihre pharmazeutisch verwendbaren Derivate, Solvate und Stereoisomere, einschließlich deren Mischungen in allen Verhältnissen, sowie gegebenenfalls Träger- und/oder Hilfsstoffe.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder oder auch als Nasenspray. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und /oder mehrere weitere Wirkstoffe enthalten, z.B. ein oder mehrere Vitamine.

Die Verbindungen der Formel I und ihre physiologisch unbedenklichen Salze können bei der Bekämpfung von Krankheiten, bei denen eine Erhöhung des cGMP(cyclo-Guanosin-monophosphat)-Spiegels zu Entzündungshemmung oder -verhinderung und Muskelentspannung führt, eingesetzt werden. Besondere Verwendung können die erfindungsgemäßen Verbindungen bei der Behandlung von Krankheiten des Herz-Kreislaufsystems und zur Behandlung und/oder Therapie von Potenzstörungen beim Menschen finden.

Gegenstand der Erfindung ist die Verwendung der Verbindungen der Formel gemäß Anspruch 1 sowie deren physiologisch unbedenklichen Salze und/oder Solvate zur Herstellung eines Arzneimittels zur Behandlung von Angina, Bluthochdruck, pulmonarem Hochdruck, congestivem Herzversagen, Herzinfarkt, chronischer obstruktiver pulmonaler Krankheit (COPD), Cor pulmonale, Rechtsherzinsuffizienz, Atherosklerose, Bedingungen nale, Rechtsherzinsuffizienz, Atherosklerose, Bedingungen verminderter Durchgängigkeit der Herzgefäße, peripheren vaskulären Krankheiten, Schlaganfall, Bronchitis, allergischem Asthma, chronischem Asthma, allergischer Rhinitis, Glaucom, Irritable Bowel Syndrome, Tumoren, Niereninsuffizienz, Leberzirrhose, zur Behandlung weiblicher Sexualstörungen, Entzündungen, Osteoporose, zur Behandlung von maligner Hypertonie, Phäochromazytom, peripherer Gefäß(-verschluß)-erkrankungen, Gefäßerkrankungen, Thrombozytopenie, Ulcus pepticum, Darmbewegungsstörungen, perkutaner transluminaler Koronarangioplastie, Karotis Angioplastie, postoperativer Stenose des Koronarbypasses, Vorwehen und benigner Prostata-Hyperplasie.

Dabei werden die Substanzen in der Regel vorzugsweise in Dosierungen zwischen etwa 1 und 500 mg, insbesondere zwischen 5 und 100 mg pro Dosierungseinheit verabreicht. Die tägliche Dosierung liegt vorzugsweise zwischen etwa 0,02 und 10 mg/kg Körpergewicht. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Gegenstand der Erfindung sind ferner Arzneimittel enthaltend mindestens eine Verbindung der Formel I gemäß Anspruch 1 und/oder ihre pharmazeutisch verwendbaren Derivate, Solvate und Stereoisomere, einschließlich deren Mischungen in allen Verhältnissen, und mindestens einen weiteren Arzneimittelwirkstoff.

Gegenstand der Erfindung ist auch ein Set (Kit), bestehend aus getrennten Packungen von
(a) einer wirksamen Menge an einer Verbindung der Formel I gemäss Anspruch 1 und/oder ihrer pharmazeutisch verwendbaren Derivate, Solvate und Stereoisomere, einschließlich deren Mischungen in allen Verhältnissen, und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer Verbindung der Formel I gemäß Anspruch 1 und/oder ihrer pharmazeutisch verwendbaren Derivate, Solvate und Stereoisomere, einschließlich deren Mischungen in allen Verhältnissen,
und einer wirksamen Menge eines weiteren Arzneimittelwirkstoffs gelöst oder in Iyophylisierter Form vorliegt.

Gegenstand der Erfindung ist ferner die Verwendung von Verbindungen der Formel I gemäß Anspruch 1und/oder ihrer pharmazeutisch verwendbaren Derivate, Solvate und Stereoisomere, einschließlich deren Mischungen in allen Verhältnissen, zur Herstellung eines Arzneimittels zur Behandlung von Krankheiten des Herz-Kreislaufsystems, zur Behandlung und/oder Therapie von Potenzstörungen, zur Behandlung von Angina, Bluthochdruck, pulmonarem Hochdruck, congestivem Herzversagen, Herzinfarkt, chronischer obstruktiver pulmonaler Krankheit (COPD), Cor pulmonale, Rechtsherzinsuffizienz, Atherosklerose, Bedingungen verminderter Durchgängigkeit der Herzgefäße, peripheren vaskulären Krankheiten, Schlaganfall, Bronchitis, allergischem Asthma, chronischem Asthma, allergischer Rhinitis, Glaucom, Irritable Bowel Syndrome, Tumoren, Niereninsuffizienz, Leberzirrhose, zur Behandlung weiblicher Sexualstörungen, Entzündungen, Osteoporose, weiterhin zur Behandlung von maligner Hypertonie, Phäochromazytom (katecholaminproduzierender Tumor der Nebennierenrinde), bei peripheren Gefäß(-verschluß)-erkrankungen, Gefäßerkrankungen, Thrombozytopenie, Ulcus pepticum (gutartiges Darmgeschwür), Darmbewegungsstörungen, perkutaner störungen, perkutaner transluminaler Koronarangioplastie, Karotis Angioplastie, postoperativer Stenose des Koronarbypasses, Vorwehen sowie benigner Prostata-Hyperplasie, in Kombination mit mindestens einem weiteren Arzneimittelwirkstoff.

Die erfindungsgemäßen Verbindungen der Formel I gemäß Anspruch 1 können zusammen mit anderen Wirkstoffen verwendet werden, wie z.B. mit Vasodilatoren, α-adrenergen Inhibitoren, wie z.B. Phentolamin, Prazocin oder Yohimbin, gemischten α,β-Inhibitoren, wie z.B. Carvedilol, Prostaglandin El und Prostacyclin, ACE (Angiotensin Converting Enzyme) - Hemmern, NEP (Neutrale Endopeptidase) - Hemmern, zentral wirksamen dopaminergen Wirkstoffen, wie z.B. Apomorphin, vasoaktiven Intestinalpeptiden, Calciumkanalblockern und Verbindungen wie Thiaziden.

Gegenstand der Erfindung sind daher pharmazeutische Formulierungen enthaltend ein Prostaglandin oder Prostaglandinderivat und mindestens eine Verbindung der Formel I gemäß Anspruch 1.
Bevorzugt sind Prostaglandine oder Prostaglandinderivate ausgewählt aus der Gruppe PGE₀, PGA₁, PGB₁, PGF_{1α}, PGA₂, PGB₂, 19-Hydroxy-PGA₁, 19-Hydroxy-PGB₁, 19-Hydroxy-PGA₂, 19-Hydroxy-PGB₂, PGE₃, PGF_{3α}, Alprostadil (PGE₁), Dinoprost (PGF₂), Dinoprostone (PGE₂), Epoprostenol Natrium (PGI₂; Prostacyclin Natrium), Gemeprost, Iloprost, Latanoprost, Misoprostol, Sulprostone, Carboprost Thromethamin, Dinoprost Thromethamin, Lipoprost, Metenoprost, Tiaprost.

Bevorzugt sind besonders Prostaglandine oder Prostaglandinderivate ausgewählt aus der Gruppe Alprostadil (PGE₁), Dinoprost (PGF₂), Dinoprostone (PGE₂), Epoprostenol Natrium (PGl₂; Prostacyclin Natrium), Gemeprost, Iloprost, Latanoprost, Misoprostol, Sulprostone, Carboprost Thromethamin, Dinoprost Thromethamin, Lipoprost, Metenoprost, Tiaprost.

Besonders bevorzugt ist PGE₁ oder Prostacyclin, insbesondere bevorzugt ist Prostacyclin.

Gegenstand der Erfindung sind ferner pharmazeutische Formulierungen enthaltend einen Calcium-Antagonisten und mindestens eine Verbindung der Formel I gemäß Anspruch 1.
Bevorzugt sind Calcium-Antagonisten ausgewählt aus der Gruppe der selektiven und nicht-selektiven Calcium-Antagonisten.

Bevorzugt sind selektive Calcium-Antagonisten ausgewählt aus der Gruppe der Dihydropyridinderivate, Phenylalkylaminderivate, Benzothiazepinderivate und anderen selektiven Calcium-Antagonisten.

Dihydropyridinderivate sind vorzugsweise ausgewählt aus der Gruppe Amlodipine, Felodipine, Isradipine, Nicardipine, Nifedipine, Nimodipine, Nisoldipine, Nitrendipine, Lacidipine, Nilvadipine, Manidipine, Bamidipine, Lercanidipine.

Die Phenylalkylaminderivate sind vorzugsweise ausgewählt aus der Gruppe Verapamil, Gallopamil.

Die Benzothiazepinderivate bedeuten vorzugsweise Diltiazem.

Die anderen selektiven Calcium-Antagonisten bedeuten vorzugsweise Mibefradil.

Die nicht-selektiven Calcium-Antagonisten sind vorzugsweise ausgewählt aus der Gruppe Fendiline, Bepridil, Lidoflazine, Perhexiline.

Gegenstand der Erfindung sind ferner pharmazeutische Formulierungen enthaltend ein Antithromboticum und mindestens eine Verbindung der Formel I gemäß Anspruch 1.

Unter den Begriff Antithrombotica fallen auch sogenannte Antikoagulantien und Blutplättchenaggregationshemmer (Thrombozytenaggregationshemmer).
Bevorzugte Antithrombotica sind Vitamin K Antagonisten, Heparinverbindungen, Thrombozytenaggregationshemmer, Enzyme, Faktor Xa Inhibitoren, Faktor VIIa Inhibitoren und andere antithrombotische Agenzien.

Bevorzugte Vitamin K Antagonisten sind ausgewählt aus der Gruppe Dicoumarol, Phenindione, Warfarin, Phenprocoumon, Acenocoumarol, Ethyl-biscoumacetat, Clorindione, Diphenadione, Tioclomarol.

Bevorzugte Heparinverbindungen sind ausgewählt aus der Gruppe Heparin, Antithrombin III, Dalteparin, Enoxaparin, Nadroparin, Parnaparin, Reviparin, Danaparoid, Tinzaparin, Sulodexide.

Bevorzugte Thrombozytenaggregationshemmer sind ausgewählt aus der Gruppe Ditazole, Cloricromen, Picotamide, Clopidogrel, Ticlopidine, Acetylsalicylsäure, Dipyridamole, Calcium carbassalat, Epoprostenol, Indobufen, Iloprost, Abciximab, Tirofiban, Aloxiprin, Intrifiban.

Bevorzugte Enzyme sind ausgewählt aus der Gruppe Streptokinase, Alteplase, Anistreplase, Urokinase, Fibrinolysin, Brinase, Reteplase, Saruplase.

Bevorzugte Antithrombotica sind weiterhin die Blutplättchen-Glycoprotein-Rezeptor (IIb/IIIa)-Antagonisten, die die Blutplättchenaggregation inhibieren.
Bevorzugte Verbindungen sind z.B. beschrieben in EP 0 623 615 B1 auf Seite 2 oder in der EP 0 741 133 A2 Seite 2, Zeile 2 bis Seite 4 Zeile 56.

Bevorzugte Faktor Xa- und VIIa-Inhibitoren sind z.B.
die in WO 9916751, WO 9931092, WO 9957096, WO 0012479, WO 0020416, WO 0040583, WO 0051989 beschriebenen Verbindungen der Formel I.
Andere bevorzugte Faktor Xa Inhibitoren sind z.B. die in den nachstehenden Dokumenten beschriebenen Verbindungen:
a) in WO 97/30971, Seite 4, Zeile 5 bis Seite 13, Zeile 19;
b) in EP 0 921 116 A1, Seite 2, Zeile 1 bis Zeile 51;
c) in EP 0 540 051 B1, Seite 2, Zeile 41 bis Seite 3, Zeile 14;
d) in EP 0 798 295 A1, Seite 69, Zeile 10 bis Seite 71, Seite 53;

Bevorzugte andere Verbindungen sind ausgewählt aus der Gruppe Defibrotide, Desirudin oder Lepirudin.

Gegenstand der Erfindung sind auch solche pharmazeutischen Formulierungen enthaltend einen Endothelin-Rezeptor-Antagonisten und mindestens eine Verbindung der Formel I gemäß Anspruch 1.

Bevorzugte Endothelin-Rezeptor-Antagonisten sind Bosentan, Tezosentan und Sitaxentan (TBC-11251; J.Med.Chem., 40, No.11, 1690-97, 1997). Bevorzugte Endothelin-Rezeptor-Antagonisten sind somit weiterhin
a) BMS-193884 (EP 558258),
b) BMS-207940 (Pharmaprojects (13.06.97)),
c) BQ-123 (Exp.Opin.Invest.Drugs, 1997, 6, No.5, 475-487),
d) SB-209670 (Exp.Opin.Invest.Drugs, 1997, 6, No.5, 475-487),
e) SB-217242 (Exp.Opin.Invest.Drugs, 1997, 6, No.5, 475-487),
f) SB-209598 (Trends in Pharmacol. Sci., 17, 177-81,1996),
g) TAK-044 (Exp.Opin.Invest.Drugs, 1997, 6, No.5, 475-487),
h) Bosentan (Trends in Pharmacol. Sci., 18, 408-12, 1997),
i) PD-156707 (J.Med.Chem., 40, No.7, 1063-74, 1997),
j) L-749329 (Bioorg.Med.Chem.Lett., 7, No.3, 275-280, 1997),
k) L-754142 (Exp.Opin.Invest.Drugs, 1997, 6, No.5, 475-487),
l) ABT-627 (J.Med.Chem., 40, No.20, 3217-27,1997),
m) A-127772 (J.Med.Chem., 39, No.5, 1039-1048, 1996),
n) A-206377 (213th American Chemical Society National Meeting, San Francisco, California, USA, 13 - 17 April 1997, Poster, MEDI 193),
o) A-182086 (J.Med.Chem., 40, No.20, 3217-27, 1997),
p) EMD-93246 (211th American Chemical Society National Meeting, New Orleans, USA, 1996, Poster, MEDI 143),
q) EMD-122801 (Bioorg.Med.Chem.Lett., 8, No.1, 17-22, 1998),
r) ZD-1611 (Trends in Pharmacol. Sci., 18, 408-12, 1997),
s) AC-610612 (R&D Focus Drug News (18.05.98)),
t) T-0201 (70th Annual Meeting of the Japanese Pharmacological Society, Chiba, Japan, 22-15 March 1997, Lecture, O-133),
u) J-104132 (R&D Focus Drug News (15.12.97)),
v) 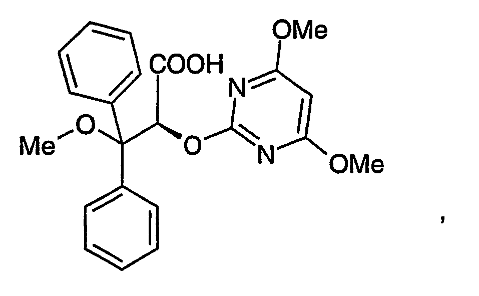
w) 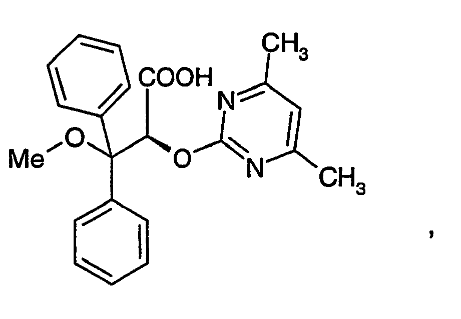
x) 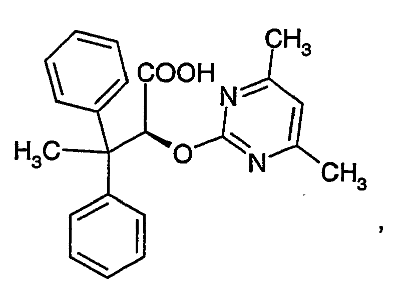
y)

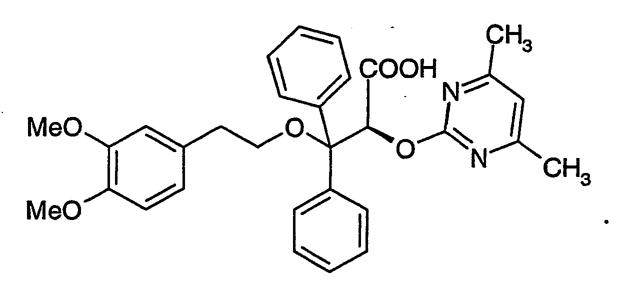

Besonders bevorzugte Endothelin-Rezeptor-Antagonisten sind z.B. die in EP 0733626, EP 0733626, EP 0755934, EP 0757039, EP 0796250, WO 9719077, WO 9730982, WO 9730996, DE 19609597, DE 19612101, WO 9827091, WO 9827077, WO 9841515, WO 9841521, WO 9842702, WO 9842709 oder WO 9905132 beschriebenen Verbindungen der Formel I.

Gegenstand der Erfindung sind weiterhin pharmazeutischen Formulierungen enthaltend einen Vasodilator, wie z.B. ein Nitrat, und mindestens eine Verbindung der Formel I gemäß Anspruch 1.

Gegenstand der Erfindung sind vorzugsweise pharmazeutische Formulierungen enthaltend mindestens eine Verbindung der Formel I gemäß Anspruch 1 und einen Vasodilator, wie z.B. (a) ein organisches Nitrat, z.B. Nitroglycerin, Isosorbidmononitrat, Isosorbiddinitrat, Pentaerythrityltetra-, Pentaerythrityltri-, Pentaerythrityldi-, Pentaerythritylmononitrat, Propatylnitrat, Trolnitrat, Nicroandil, Mannitolhexanitrat, Inositolhexanitrat, N-[3-Nitratopivaloyl]-L-cycsteinethylester, (b) ein organisches Nitrit, z.B. Isoamylnitrit, (c) ein Thionitrit, (d) ein Thionitrat, (e) ein S-Nitrosothiol, wie z.B. S-Nitroso-N-acetyl-D,L-penicillamin, (f) Nitrosoproteine, (g) substituierte Furoxane, wie z.B. 1,2,5-Oxadiazol-2-oxide oder Furazan-N-oxide, (h) substituierte Sydnonimine, wie z.B. Molsidomine oder Mesocarb, (i) komplexe Nitrosylverbindungen, wie z.B. Eisennitrosylverbindungen, vorzugsweise Natriumnitroprussid oder (j) Stickoxid NO, das inhaliert wird.

Bevorzugte Vasodilatoren sind Nitrate ausgewählt aus der Gruppe Pentaerythrityltetra-, Pentaerythrityltri-, Pentaerythrityldi-, Pentaerythritylmononitrat, Isosorbidmononitrat, Isosorbiddinitrat, Glyceroltrinitrat.

Bevorzugt sind besonders Nitrate ausgewählt aus der Gruppe Pentaerythrityltetranitrat, Isosorbidmononitrat, Isosorbiddinitrat, Glyceroltrinitrat, ganz besonders bevorzugt ist Pentaerythrityltetranitrat.

Gegenstand der Erfindung ist weiterhin die Verwendung einer pharmazeutischen Formulierung enthaltend mindestens eine Verbindung der Formel I gemäß Anspruch 1 und mindestens ein Antithromboticum zur Herstellung eines Arzneimittels zur Behandlung von pulmonalem Hochdruck, congestivem Herzversagen (CHF), chronischer obstruktiver pulmonaler Krankheit (COPD), Cor pulmonale und/oder Rechtsherzinsuffizienz.

α-Adrenerge Inhibitoren hemmen die Vasokonstriktion im Corpus Cavernosum. Da PDE V - Hemmer die Vasodilatation des gleichen Gewebes der glatten Muskulatur erhöht, können zur Behandlung von Potenzstörungen (erektiler Dysfunktion) vorzugsweise auch pharmazeutische Formulierungen verwendet werden, enthaltend mindestens eine Verbindung der Formel I gemäß Anspruch 1 und mindestens einen α-adrenergen Inhibitor, wie z.B. Phentolamin oder Prazocin oder mindestens einen zentral wirksamen dopaminergen Wirkstoff, wie z.B. Apomorphin.

Gegenstand der Erfindung ist daher weiterhin die Verwendung einer pharmazeutischen Formulierung enthaltend mindestens eine Verbindung der Formel I gemäß Anspruch 1 und mindestens einen α-adrenergen Inhibitor, wie z.B. Phentolamin oder Prazocin oder mindestens einen zentral wirksamen dopaminergen Wirkstoff, wie z.B. Apomorphin zur Herstellung eines Arzneimittels zur Behandlung von Potenzstörungen.

Gegenstand der Erfindung ist weiterhin die Verwendung einer pharmazeutischen Formulierung enthaltend mindestens eine Verbindung der Formel I gemäß Anspruch 1 und mindestens einen Calcium-Antagonisten zur Herstellung eines Arzneimittels zur Behandlung von pulmonalem Hochdruck, congestivem Herzversagen (CHF), chronischer obstruktiver pulmonaler Krankheit (COPD), Cor pulmonale und/oder Rechtsherzinsuffizienz.

Gegenstand der Erfindung ist weiterhin die Verwendung einer pharmazeutischen Formulierung enthaltend mindestens eine Verbindung der Formel I gemäß Anspruch 1 und mindestens ein Nitrat zur Herstellung eines Arzneimittels zur Behandlung von pulmonalem Hochdruck, congestivem Herzversagen (CHF), chronischer obstruktiver pulmonaler Krankheit (COPD), Cor pulmonale und/oder Rechtsherzinsuffizienz.

Gegenstand der Erfindung ist weiterhin die Verwendung einer pharmazeutischen Formulierung enthaltend mindestens eine Verbindung der Formel I gemäß Anspruch 1 und mindestens einen Endothelin-Rezeptor-Antagonisten zur Herstellung eines Arzneimittels zur Behandlung von pulmonalem Hochdruck, congestivem Herzversagen (CHF), chronischer obstruktiver pulmonaler Krankheit (COPD), Cor pulmonale und/oder Rechtsherzinsuffizienz.

Gegenstand der Erfindung ist weiterhin die Verwendung einer pharmazeutischen Formulierung enthaltend mindestens eine Verbindung der Formel I gemäß Anspruch 1 und mindestens ein Prostaglandin oder ein Prostaglandinderivat zur Herstellung eines Arzneimittels zur Behandlung von pulmonalem Hochdruck, congestivem Herzversagen (CHF), chronischer obstruktiver pulmonaler Krankheit (COPD), Cor pulmonale und/oder Rechtsherzinsuffizienz.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation.
Massenspektrometrie (MS): EI (Elektronenstoß-lonisation) M⁺
FAB (Fast Atom Bombardment) (M+H)⁺

### Beispiel 1

### Herstellung von 4-[4-(3-Chlor-4-methoxy-benzylamino)-9-(1-phenyl-ethyl)-6,7,8,9-tetrahydro-5H-1,3,9-triaza-fluoren-2-yl]-benzoesäure

1.1 Eine Lösung von 56,6 g 2-Hydroxycyclohexanon, 60,6 g Phenethylamin und einer katalytischen Menge p-Toluolsulfonsäure in 400 ml Cyclohexanon wird 10 Stunden am Wasserabscheider unter Rückfluß gekocht. Die Lösung wird auf 50° abkühlt, dann werden 10 ml Piperidin und 30 ml Malonsäuredinitril, gelößt in heißem Cyclohexanon, zugegeben und es wird weiter einige Stunden unter Rückfluß gekocht.
Nach Abtrennung des Lösungsmittels und üblicher Aufarbeitung erhält man 45 g 2-Amino-1-(1-phenyl-ethyl)-4,5,6,7-tetrahydro-1*H*-indol-3-carbonitril ("AA"), F. 129°,

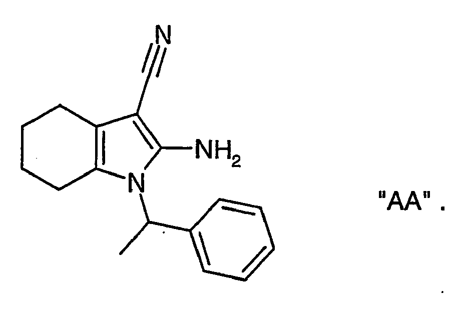

1.2 1,8 g 4-N,N-Dimethylaminocarbonyl-benzoesäuremethylester wird unter Eiskühlung mit 1 ml POCl₃ versetzt und ca. 30 Minuten nachgerührt. Man gibt 2 g "AA" zu und rührt das Gemisch 3 Stunden bei 80°. Danach wird wie üblich aufgearbeitet und man erhält 1,6 g 4-[4-Chlor-9-(1-phenylethyl)-6,7,8,9-tetrahydro-5*H*-1,3,9-triaza-fluoren-2-yl]-benzoesäuremethylester ("AB"),

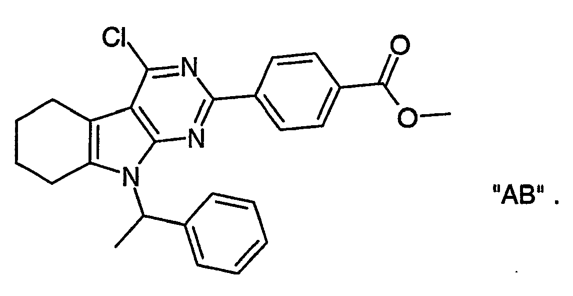

1.3 Eine Lösung von 1,6 g "AB" in 30 ml 1-Methyl-2-pyrrolidon wird mit 3-Chlor-4-methoxy-benzylamin versetzt und 4 Stunden bei 100° gerührt. Man arbeitet wie üblich auf und erhält 1,4 g 4-[4-(3-Chlor-4-methoxybenzylamino)-9-(1-phenyl-ethyl)-6,7,8,9-tetrahydro-5*H*-1,3,9-triaza-fluoren-2-yl]-benzoesäuremethylester ("AC").

1.4 0,5 g "AC" wird in 20 ml Ethylenglycolmonoethylether vorgelegt, mit 20 ml NaOH versetzt und 3 Stunden am Dampfbad erhitzt. Der Ether wird abgetrennt, der Rückstand mit Wasser verdünnt und mit Ethylacetat gewaschen. Das Ethylacetat wird verworfen. Es wird mit Eisessig angesäuert und das ausgefallene Kristallisat wird abgetrennt. Der Rückstand wird in Methanol gelöst und mit methanolischer KOH versetzt. Der Methanol wird im Vakuum abgetrennt. Der Rückstand wird mit Wasser versetzt und das ausgefallene Kristallisat wird abgesaugt. Man erhält 0,11 g 4-[4-(3-Chlor-4-methoxy-benzylamino)-9-(1-phenyl-ethyl)-6,7,8,9-tetrahydro-5*H-*1,3,9-triaza-fluoren-2-yl]-benzoesäure, Kaliumsalz, F. 179°.

Affinität zu PDE V: IC₅₀ [mol/l] 3.0E-07

### Beispiel 2

### Herstellung von 4-[4-(3,4-Methylendioxy-benzylamino)-5,6,7,8-tetrahydrobenzo[4,5]furo[2,3-d]pyrimidin-2-yl]-benzoesäure

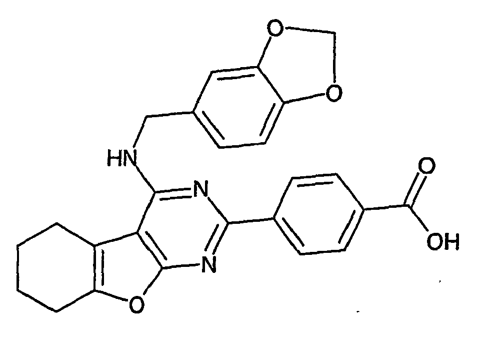

2.1 Zu einer Lösung von 11,0 g 2-Hydroxycyclohexanon und 9,6 g Malonsäuredinitril in 25 ml Methanol tropft man 4,8 ml Triethylamin und rührt das Gemisch 4 Stunden bei Raumtemperatur nach. Das ausgefallene Kristallisat wird abgetrennt und mit Methanol gewaschen.
Man erhält 12,8 g 2-Amino-4,5,6,7-tetrahydro-benzofuran-3-carbonitril ("BA"), F. 179°.

2.2 30 ml einer 40%igen Dimethylaminlösung wird in 200 ml THF vorgelegt. 20 g 4-Chlorcarbonyl-benzoesäuremethylester werden in 300 ml THF gelöst und zugetropft und das Gemisch wird 2 Stunden bei Raumtemperatur nachgerührt. Das Lösungsmittel wird entfernt und es wird wie üblich aufgearbeitet. Man erhält 19,2 g 4-(N,N-Dimethylaminocarbonyl)-benzoesäuremethylester ("BB"), F. 107°.

2.3 5,0 g "BB" wird unter Eiskühlung mit 3,3 ml POCl₃ versetzt und ca. 30 Minuten nachgerührt. Dann werden 5,9 g "BA" zugegeben und das Gemisch wird 3 Stunden bei 80° gerührt.
Nach üblicher Aufarbeitung erhält man 5,2 g 4-(4-Chlor-5,6,7,8-tetrahydrobenzo[4,5]furo[2,3-*d*]pyrimidin-2-yl)-benzoesäuremethylester ("BC"), amorph.

2.4 Eine Lösung von 2,5 g "BC" in 30 ml 1-Methyl-2-pyrrolidon wird mit 2,4 g 3,4-Methylendioxy-benzylamin versetzt und 4 Stunden bei 100° gerührt. Man arbeitet wie üblich auf und erhält 2,2 g 4-[4-(3,4-Methylendioxybenzylamino)-5,6,7,8-tetrahydro-benzo[4,5]furo[2,3-*d*]pyrimidin-2-yl]-benzoesäuremethylester ("BD").

2.5 1,0 g "BD" wird in 10 ml Ethylenglycolmonoethylether vorgelegt, mit 10 ml NaOH versetzt und 3 Stunden am Dampfbad erhitzt. Der Ether wird abgetrennt, der Rückstand mit Wasser verdünnt und mit Ethylacetat gewaschen. Das Ethylacetat wird verworfen. Es wird mit Eisessig angesäuert und das ausgefallene Kristallisat wird abgetrennt. Der Rückstand wird in Isopropanol gelöst und mit Ethanolamin versetzt. Das Salz wird ausgeäthert und man erhält 0,5 g 4-[4-(3,4-Methylendioxy-benzylamino)-5,6,7,8-tetrahydro-benzo[4,5]furo[2,3-*d*]pyrimidin-2-yl]-benzoesäure, Ethanolaminsalz, F. 205°.

Analog erhält man durch Umsetzung von
4-(4-Chlor-5,6,7,8-tetrahydro-benzo[4,5]furo[2,3-*d*]pyrimidin-2-yl)-benzoesäuremethylester
mit
3-Chlor-4-methoxy-benzylamin,
Benzylamin,
3,4-Dimethoxy-benzylamin,
3,4-Dichlor-benzylamin,
C-Pyridin-3-yl-methylamin
und nachfolgender Esterhydrolyse
4-[4-(3-Chlor-4-methoxy-benzylamino)-5,6,7,8-tetrahydrobenzo[4,5]furo[2,3-*d*]pyrimidin-2-yl]-benzoesäure, Ethanolaminsalz, F. 205°;
4-(4-Benzylamino-5,6,7,8-tetrahydro-benzo[4,5]furo[2,3-*d*]pyrimidin-2-yl)-benzoesäure, Ethanolaminsalz;
4-[4-(3,4-Dimethoxy-benzylamino)-5,6,7,8-tetrahydrobenzo[4,5]furo[2,3-*d*]pyrimidin-2-yl]-benzoesäure, Ethanolaminsalz;
4-[4-(3,4-Dichlor-benzylamino)-5,6,7,8-tetrahydro-benzo[4,5]furo[2,3-*d*]pyrimidin-2-yl]-benzoesäure, Ethanolaminsalz;
4-{4-[(Pyridin-3-ylmethyl)-amino]-5,6,7,8-tetrahydro-benzo[4,5]-furo[2,3-*d*]pyrimidin-2-yl]-benzoesäure, Ethanolaminsalz, F. >250°.

### Beispiel 3

### Herstellung von Herstellung von 4-[4-(3-Chlor-4-methoxy-benzylamino)-5,6,7,8-tetrahydro-benzo[4,5]furo[2,3-d]pyrimidin-2-yl]-benzoesäure

3.1. 5,5 g 4-(N,N-Dimethylcarbamoyl)-buttersäuremethylester wird unter Eiskühlung mit 3,3 ml POCl₃ versetzt und ca. 30 Minuten nachgerührt. Dann werden 5,9 g "BA" zugegeben und das Gemisch wird 3 Stunden bei 80° gerührt.
Nach üblicher Aufarbeitung erhält man 5,2 g 4-(4-Chlor-5,6,7,8-tetrahydrobenzo[4,5]furo[2,3-*d*]pyrimidin-2-yl)-buttersäuremethylester ("CA"), amorph.

3.2 Eine Lösung von 2,0 g "CA" in 20 ml 1 -Methyl-2-pyrrolidon wird mit 2,3 g 3-Chlor-4-methoxy-benzylamin versetzt und 3 Stunden bei 80° gerührt. Man arbeitet wie üblich auf und erhält 2,2 g 4-[4-(3-Chlor-4-methoxybenzylamino)-5,6,7,8-tetrahydro-benzo[4,5]furo[2,3-*d*]pyrimidin-2-yl]-buttersäuremethylester ("CB").

3.3 0,8 g "CB" wird in 10 ml Methanol vorgelegt, mit 10 ml 2N NaOH versetzt und 2 Stunden bei 50° nachgerührt. Der Alkohol wird abgetrennt, der Rückstand mit Wasser verdünnt und mit Ethylacetat gewaschen. Das Ethylacetat wird verworfen. Es wird mit HCl angesäuert und wie üblich aufgearbeitet. Der Rückstand wird in Ethanol gelöst und mit Cyclohexylamin versetzt. Das ausgefallene Kristallisat wird mit Ether gewaschen und man erhält 0,25 g 4-[4-(3-Chlor-4-methoxy-benzylamino)-5,6,7,8-tetrahydrobenzo[4,5]furo[2,3-*d*]pyrimidin-2-yl]-buttersäure, Cyclohexylaminsalz, F. 205°.

### Beispiel 4

### Herstellung von 1-[4-(3-Chlor-4-methoxy-benzylamino)-5,6,7,8-tetrahydrobenzo[4,5]furo[2,3-d]pyrimidin-2-ylmethyl]-piperidin-4-carbonsäure

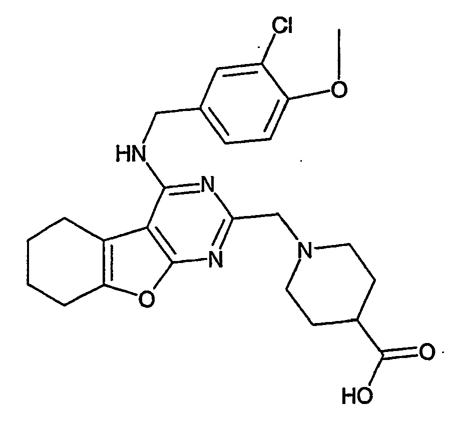

4.1 Zu 6,2 ml 2-Chlordimethylacetamid tropft man unter Rühren und Kühlen 6,7 ml POCl₃ und rührt 10 Minuten bei Raumtemperatur nach. Man gibt 10,0 g "BA" zu und rührt 30 Minuten bei 80° nach. Nach üblicher Aufarbeitung erhält man 8,3 g 4-Chlor-2-chlormethyl-5,6,7,8-tetrahydrobenzo[4,5]furo[2,3-*d*]pyrimidin ("DA") als Öl.

4.2 Zu einer Lösung von 8,3 g "DA" in 100 ml THF gibt man 10 ml Piperidin-4-carbonsäureethylester und rührt 16 Stunden bei Raumtemperatur nach.
Man gibt Wasser zu und extrahiert mit Methyl-tert.-butylether (MTB). Der MTB-Extrakt ("X") wird mit verdünnter HCl extrahiert.

Der HCl-Extrakt wird mit verdünnter NaOH alkalisiert, mit MTB extrahiert und wie üblich aufgearbeitet. Man erhält 2,9 g 1-(4-Chlor-5,6,7,8-tetrahydro-benzo[4,5]furo[2,3-*d*]pyrimidin-2-ylmethyl)-piperidin-4-carbonsäureethylester ("DB").

"X" enthält das Nebenprodukt 1-(2-Chlormethyl-5,6,7,8-tetrahydrobenzo[4,5]furo[2,3-d]pyrimidin-4-yl)piperidin-4-carbonsäureethylester.

4.3 Eine Lösung von 2,9 g "DB" in 50 ml 1-Methyl-2-pyrrolidon wird mit 3,0 g 3-Chlor-4-methoxy-benzylamin versetzt und 1 Stunde über dem Dampfbad gerührt. Man arbeitet wie üblich auf und erhält 2,9 g 1-[4-(3-Chlor-4-methoxy-benzylamino)-5,6,7,8-tetrahydro-benzo[4,5]furo[2,3-*d*]pyrimidin-2-ylmethyl]-piperidin-4-carbonsäureethylester ("DC"), F. 152°.

4.4 2,8 g "DC" wird in 30 ml Ethylenglycolmonoethylether vorgelegt, mit 3 ml NaOH (ca. 30%ig) versetzt und 1 Stunde bei 60° nachgerührt. Der Ether wird abgetrennt und mit Essigsäure auf pH 4 angesäuert. Das ausgefallene Kristallisat wird mit Eiswasser gewaschen und man erhält 1,5 g 1-[4-(3-Chlor-4-methoxy-benzylamino)-5,6,7,8-tetrahydro-benzo[4,5]-furo[2,3-*d*]pyrimidin-2-ylmethyl]-piperidin-4-carbonsäure, F. 233-235°.

### Beispiel 5

### Herstellung von 1-[4-(3-Chlor-4-methoxy-benzylamino)-5,6,7,8-tetrahydrobenzo[4,5]furo[2,3-d]pyrimidin-2-ylmethyl]-4-methylsulfonyl-piperazin

5.1 Zu einer Lösung von 4,7 g "DA" und 1,8 g Triethylamin Puffersubstanz f in 50 g Dichlormethan tropft man 2,85 g Piperazin-N-carbonsäureethylester und rührt 16 Stunden nach. Man arbeitet wie üblich auf und erhält 3,0 g 1-(4-Chlor-5,6,7,8-tetrahydro-benzo[4,5]furo[2,3-*d*]pyrimidin-2-ylmethyl)-4-ethoxycarbonyl-piperazin ("EA") als Öl.

5.2 Eine Lösung von 3,4 g "EA" in 80 ml 1,4-Dioxan wird mit 3,49 g 3-Chlor-4-methoxy-benzylamin versetzt und 16 Stunden bei 115° gerührt. Die ausgefallenen Kristalle und das Lösungsmittel werden abgetrennt. Der Rückstand wird mit 10 ml 1-Methyl-2-pyrrolidon versetzt und 1,5 Stunden bei 115° gerührt. Man arbeitet wie üblich auf und erhält 3,25 g 1-[4-(3-Chlor-4-methoxy-benzylamino)-5,6,7,8-tetrahydro-benzo[4,5]furo[2,3-*d*]pyrimidin-2-ylmethyl]-4-ethoxycarbonyl-piperazin ("EB").

5.3 Zu einer Lösung von 3,25 g "EB" in 20 ml Ethylenglycolmonoethylether gibt man 3,5 ml 32 %ige NaOH und rührt 5 Stunden bei 110° nach. Nach üblicher Aufarbeitung erhält man 1,99 g 1-[4-(3-Chlor-4-methoxybenzylamino)-5,6,7,8-tetrahydro-benzo[4,5]furo[2,3-*d*]pyrimidin-2-ylmethyl]-piperazin ("EC") als Öl.

5.4 Zu einer Lösung von 2,0 g "EC" und 360 mg Pyridin in 50 ml Dichlormethan tropft man 520 mg Methansulfonylchlorid und rührt 3 Stunden bei Raumtemperatur nach. Nach üblicher Aufarbeitung erhält man 1,0 g 1-[4-(3-Chlor-4-methoxy-benzylamino)-5,6,7,8-tetrahydro-benzo[4,5]-furo[2,3-*d*]pyrimidin-2-ylmethyl]-4-methylsulfonyl-piperazin, Dihydrochlorid.

### Beispiel 6

### Herstellung von 4-[4-(3-Chlor-4-methoxy-benzylamino)-9H-1,3,9-triazafluoren-2-yl]-buttersäure

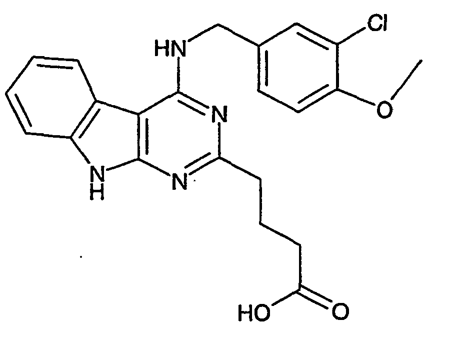

6.1 In 350 ml kaltes THF leitet man 30 Minuten Ammoniak ein. Dann werden 30 g 4-Chlorcarbonyl-buttersäuremethylester zugetropft, wobei gleichzeitig weiterhin Ammoniak eingeleitet wird. Die ausgefallenen Kristalle werden abgetrennt und der Rückstand wird mit Ethylacetat mehrmals ausgekocht. Die vereinigten Filtrate werden abdestilliert und der Rückstand wird aus Ethylacetat umkristallisiert.
Man erhält 23,4 g 4-Aminocärbonyl-buttersäuremethylester ("FA"), F. 80°.

6.2 In eine Lösung von 15 ml DMF in 300 ml Acetonitril werden bei -2 bis 0° 15 ml Oxalylchlorid zugetropft und 30 Minuten nachgerührt. Man tropft bei max. 0° 23,25 g "FA" zu und nach 15 Minuten Rühren 29 ml Pyridin. Man gibt Ether zu und arbeitet wie üblich auf.
Man erhält 20 g 4-Cyan-buttersäuremethylester ("FB") als Öl.

6.3 Zu einer Lösung von 88 g NaH-Suspension in 2 Liter DMF tropft man bei 0-5° unter Rühren und Stickstoffatmosphäre eine Lösung von 180 ml Methyl-cyanacetat in 300 ml DMF. Man rührt 30 Minuten ohne Kühlung nach. Anschließend wird eine Lösung von 106 ml o-Fluornitrobenzol in 100 ml DMF zugetropft und 14 Stunden bei Raumtemperatur nachgerührt. Man säuert mit 10 %iger HCl an, arbeitet wie üblich auf und erhält 165 g Cyan-(2-nitrophenyl)-essigsäuremethylester ("FC"), F. 58-60°.

6.4 Eine Lösung von 74 g "FC" in 180 ml Essigsäure und 550 ml Toluol wird auf 80° erhitzt. Man gibt unter Rühren und unter Beachtung der Temperatur (80 - 85°) portionsweise 130 g Zinkpulver zu und rührt 14 Stunden bei Raumtemperatur nach. Nach üblicher Aufarbeitung erhält man 30 g 2-Amino-3-methoxycarbonyl-indol ("FD"), F. 136°.

6.5 In eine Lösung von 4,0 g "FD" und 2,8 g "FB" in 60 ml 1,4-Dioxan leitet man unter Rühren und bei max. 30° 2 Stunden lang HCl ein. Die Lösung bleibt 48 Stunden stehen. Nach üblicher Aufarbeitung erhält man 1,4 g 2-[(4-Methoxycarbonyl-butyrimidoyl)-amino]-1H-indole-3-carbonsäuremethylester ("FE"), F. 95°,

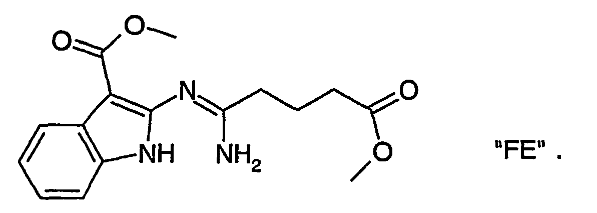

6.6 Man gibt 1,4 g "FE" in 50 ml Essigsäure, rührt 1 Stunde im Dampfbad, arbeitet wie üblich auf und erhält 1,0 g 4-(4-Oxo-4,9-dihydro-3*H*-1,3,9-triaza-fluoren-2-yl)-buttersäuremethylester ("FF"), F. 258°,

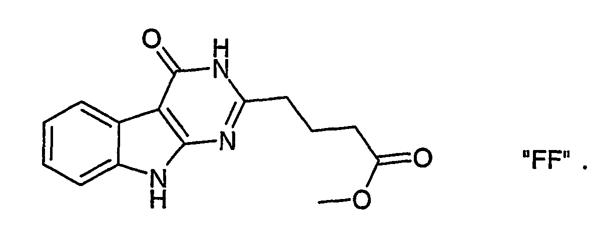

6.7 1,0 g "FF" wird mit 40 ml POCl₃ 3 Stunden unter Rückfluß gekocht. Nach Entfernung des POCl₃ wird der Rückstand 2x mit Dichlormethan behandelt. Man erhält 1,0 g 4-(4-Chlor-9*H*-1,3,9-triaza-fluoren-2-yl)-buttersäuremethylester ("FG"), F. 258°,

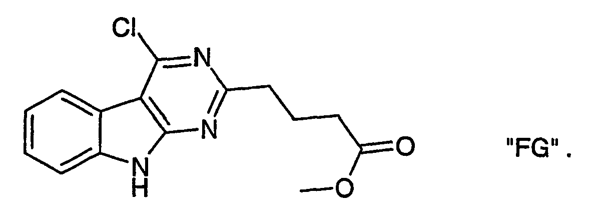

6.8 Eine Lösung von 1,0 g "FG" und 1,0 g 3-Chlor-4-methoxybenzylamin in 30 ml 1-Methyl-2-pyrrolidon wird auf 180° erhitzt. Nach Entfernen des Lösungsmittels wird aus n-Butanol umkristallisiert. Man erhält 0,45 g 4-[4-(3-Chlor-4-methoxy-benzylamino)-9*H*-1,3,9-triaza-fluoren-2-yl]-buttersäuremethylester ("FH").

6.9 Eine Lösung von 0,35 g "FH" und 10 ml 2N NaOH in 20 ml Methanol wird 1 Stunde im Dampfbad erhitzt. Man entfernt das Methanol, gibt Wasser zu, säuert mit HCl und trennt ab. Man erhält 300 mg 4-[4-(3-Chlor-4-methoxy-benzylamino)-9*H*-1,3,9-triaza-fluoren-2-yl]-buttersäure, F. 258°. Die Säure wird in Methanol gelöst und mit 2 Tropfen Ethanolamin versetzt. Man gibt Diethylether zu und isoliert das ausgefallene Kristallisat. Man erhält 280 mg 4-[4-(3-Chlor-4-methoxy-benzylamino)-9*H*-1,3,9-triaza-fluoren-2-yl]-buttersäure, Ethanolaminsalz, F. 143°.

Analog erhält man ausgehend von 4-Cyanbenzoesäuremethylester anstelle von "FB" die nachstehende Verbindung
4-[4-(3-Chlor-4-methoxy-benzylamino)-9*H*-1,3,9-triaza-fluoren-2-yl]-benzoesäure, Natriumsalz, F. > 250°.

Analog erhält man die nachstehenden Verbindungen
4-[4-(3,4-Methylendioxy-benzylamino)-9*H*-1,3,9-triaza-fluoren-2-yl]-buttersäure,
4-(4-Benzylamino-9*H*-1,3,9-triaza-fluoren-2-yl)-buttersäure,
4-[4-(3,4-Dimethoxy-benzylamino)-9*H*-1,3,9-triaza-fluoren-2-yl]-buttersäure,
4-[4-(3,4-Dichlor-benzylamino)-9*H*-1,3,9-triaza-fluoren-2-yl]-buttersäure,
4-{4-[(Pyridin-3-ylmethyl)-amino]-9*H*-1,3,9-triaza-fluoren-2-yl}-buttersäure,
4-[4-(3-Chlor-4-methoxy-benzylamino)-9*H*-1,3,9-triaza-fluoren-2-yl]-valeriansäure,
4-[4-(3,4-Methylendioxy-benzylamino)-9*H*-1,3,9-triaza-fluoren-2-yl]-valeriansäure,
4-(4-Benzylamino-9*H*-1,3,9-triaza-fluoren-2-yl)-buttersäure,
4-[4-(3,4-Dimethoxy-benzylamino)-9*H*-1,3,9-triaza-fluoren-2-yl]-valeriansäure,
4-[4-(3,4-Dichlor-benzylamino)-9*H*-1,3,9-triaza-fluoren-2-yl]-valeriansäure,
4-{4-[(Pyridin-3-ylmethyl)-amino]-9*H*-1,3,9-triaza-fluoren-2-yl}-valeriansäure,
4-[4-(3-Chlor-4-methoxy-benzylamino)-9*H*-1,3,9-triaza-fluoren-2-yl]-cyclohexan-carbonsäure,
4-[4-(3,4-Methylendioxy-benzylamino)-9*H*-1,3,9-triaza-fluoren-2-yl]-cyclohexan-carbonsäure,
4-(4-Benzylamino-9*H*-1,3,9-triaza-fluoren-2-yl)-buttersäure,
4-[4-(3,4-Dimethoxy-benzylamino)-9*H*-1,3,9-triaza-fluoren-2-yl]-cyclohexan-carbonsäure,
4-[4-(3,4-Dichlor-benzylamino)-9*H*-1,3,9-triaza-fluoren-2-yl]-cyclohexan-carbonsäure,
4-{4-[(Pyridin-3-ylmethyl)-amino]-9*H*-1,3,9-triaza-fluoren-2-yl}-cyclohexan-carbonsäure,
4-[4-(3-Chlor-4-methoxy-benzylamino)-9*H*-1,3,9-triaza-fluoren-2-yl]-benzoesäure,
4-[4-(3,4-Methylendioxy-benzylamino)-9*H*-1,3,9-triaza-fluoren-2-yl]-benzoesäure,
4-(4-Benzylamino-9*H*-1,3,9-triaza-fluoren-2-yl)-benzoesäure,
4-[4-(3,4-Dimethoxy-benzylamino)-9*H*-1,3,9-triaza-fluoren-2-yl]-benzoesäure,
4-[4-(3,4-Dichlor-benzylamino)-9*H*-1,3,9-triaza-fluoren-2-yl]-benzoesäure,
4-{4-[(Pyridin-3-ylmethyl)-amino]-9*H*-1,3,9-triaza-fluoren-2-yl}-benzoesäure.

### Beispiel 7

### Herstellung von 4-[4-(3,4-Methylendioxy-benzylamino)-9-ethyl-9H-1,3,9-triaza-fluoren-2-yl]-benzoesäure

7.1 448 g NaH werden in 9 L DMF vorgelegt, dann werden unter starker Eiskühlung und Stickstoffatmosphäre bei 2 - 8° 967 g Cyanacetamid binnen 1 Stunde portionsweise zugegeben. Man rührt 30 Minuten nach und gibt 600 ml 1-Fluor-2-nitrobenzol zu und rührt 2 Stunden nach. Man gießt in 50 L Eiswasser, säuert mit 3 L konz. HCl an und arbeitet wie üblich weiter auf. Man erhält 956 g 2-Cyan-2-(2-nitrophenyl)-acetamid ("GA"), F. 172-174°.

7.2 Analog Beispiel 6.4 erhält man aus 956 g "GA" 352 g 2-Amino-3-aminocarbonyl-indol ("GB").

7.3 13 g NaH werden in 500 ml DMF suspendiert. Anschließend tropft man eine Lösung von 52,6 g "GB" in 200 ml DMF unter Rühren bei 0° zu. Dann werden 26 ml Iodethan in 100 ml DMF bei 0° zugetropft und es wird 1 Stunde nachgerührt. Man arbeitet wie üblich auf und erhält 67,9 g 2-Amino-3-aminocarbonyl-1-ethyl-indol ("GC").

7.4 Eine Lösung von 10,0 g "GC", 8,0 g 4-Formyl-benzoesäuremethylester und 9,4 g Natriumdisulfit in 100 ml N,N-Dimethylacetamid wird 7 Stunden bei 160° gerührt. Nach üblicher Aufarbeitung erhält man 4,8 g 4-(9-Ethyl-4-oxo-4,9-dihydro-3*H*-1,3,9-triaza-fluoren-2-yl)-benzoesäuremethylester ("GD").

7.5 Zu einer Lösung von 4,8 g "GD" in 100 ml Thionylchlorid tropft man 5 ml DMF. Der Ansatz bleibt 60 Stunden stehen. Nach üblicher Aufarbeitung erhält man 3,9 g 4-(4-Chlor-9-ethyl-9*H*-1,3,9-triaza-fluoren-2-yl)-benzoesäuremethylester ("GE").

7.6 Eine Lösung von 2,4 g "GE" und 2 ml Piperonylamin in 5 ml 1-Methyl-2-pyrrolidon wird 2 Stunden bei 110° Badtemperatur gerührt. Nach üblicher Aufarbeitung erhält man 2,7 g 4-[4-(3,4-Methylendioxy-benzylamino)-9-ethyl-9*H*-1,3,9-triaza-fluoren-2-yl]-benzoesäuremethylester ("GF").

7.7 Durch Behandlung von 2,6 g "GF" mit 5 ml NaOH (ca. 32 %ig) in 10 ml Ethylenglycolmonoethylether erhält man 2,4 g 4-[4-(3,4-Methylendioxybenzylamino)-9-ethyl-9*H*-1,3,9-triaza-fluoren-2-yl]-benzoesäure, Natriumsalz, F. > 250°.

### Beispiel 8

### Herstellung von 4-[4-(3-Chlor-4-methoxy-benzylamino)-9-methyl-9H-1,3,9-triaza-fluoren-2-yl]-benzoesäure

8.1 Eine Lösung von 38 g "GB", 36,1 g 4-Formyl-benzoesäuremethylester und 41,8 g Natriumdisulfit in 200 ml N,N-Dimethylacetamid wird 3 Stunden bei 140° gerührt. Das Produkt kristallisiert aus und man erhält nach Waschen
30 g 4-(4-Oxo-4,9-dihydro-3H-1,3,9-triaza-fluoren-2yl)-benzoesäuremethylester ("HA"), F. > 250°.

8.2 Zu einer Lösung von 10 g "HA" in 100 ml Thionylchlorid tropft man 30 ml DMF. Der Ansatz bleibt 16 Stunden stehen. Nach üblicher Aufarbeitung erhält man 8,5 g 4-(4-Chlor-9*H*-1,3,9-triaza-fluoren-2-yl)-benzoesäuremethylester ("HB").

8.3 Zu einer Lösung von 3,2 g "HB" in 50 ml DMF gibt man 400 mg NaH (Suspension), rührt 1 Stunde, gibt anschließend 2 ml Iodmethan zu und rührt 1 weitere Stunde nach. Nach üblicher Aufarbeitung erhält man 3,0 g 4-(4-Chlor-9-methyl-9*H*-1,3,9-triaza-fluoren-2-yl)-benzoesäuremethyfester ("HC").

8.4 Analog Beispiel 7.6 erhält man aus 1,5 g "HC" durch Umsetzung mit 3-Chlor-4-methoxybenzylamin 1,0 g 4-[4-(3-Chlor-4-methoxy-benzylamino)-9-methyl-9*H*-1,3,9-triaza-fluoren-2-yl]-benzoesäuremethylester ("HD"), F. 205-206°.

8.4 Durch Esterhydrolyse analog Beispiel 7.7 erhält man aus "HD" 4-[4-(3-Chlor-4-methoxy-benzylamino)-9-methyl-9*H*-1,3,9-triaza-fluoren-2-yl]-benzoesäure, Natriumsalz, F. > 250°.

Analog erhält man durch Umsetzung von "HC" mit 3,4-Methylendioxy-benzylamin und anschließender Esterhydrolyse
4-[4-(3,4-Methylendioxy-benzylamino)-9-methyl-9*H*-1,3,9-triazafluoren-2-yl]-benzoesäure, Natriumsalz, F. > 250°.

Analog erhält man durch Umsetzung von "GB" mit 5-Formylpentansäuremethylester, Chlorierung, Methylierung, Umsetzung mit 3-Chlor-4-methoxy-benzylamin und Esterhydrolyse
5-[4-(3-Chlor-4-methoxy-benzylamino)-9-methyl-9*H*-1,3,9-triazafluoren-2-yl]-pentansäure, Kaliumsalz.

Analog erhält man durch Umsetzung von "HB" mit Benzylbromid, Umsetzung mit 3-Chlor-4-methoxybenzylamin und anschließender Esterhydrolyse
4-[4-(3-Chlor-4-methoxy-benzylamino)-9-benzyl-9*H*-1,3,9-triazafluoren-2-yl]-benzoesäure, Natriumsalz, F. > 250°.

Analog erhält man durch Umsetzung von "HB" mit 2-lodpropan, Umsetzung mit 3-Chlor-4-methoxybenzylamin und anschließender Esterhydrolyse
4-[4-(3-Chlor-4-methoxy-benzylamino)-9-isopropyl-9*H*-1,3,9-triazafluoren-2-yl]-benzoesäure, Natriumsalz, F. > 250°.

Die nachfolgenden Beispiele betreffen pharmazeutische Zubereitungen:

### Beispiel A: Injektionsgläser

Eine Lösung von 100 g eines Wirkstoffes der Formel 1 und 5 g Dinatriumhydrogenphosphat wird in 31 zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert. durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

### Beispiel I: Inhalations-Spray

Man löst 14 g Wirkstoff der Formel I in 10 l isotonischer NaCl-Lösung und füllt die Lösung in handelsübliche Sprühgefäße mit Pump-Mechanismus. Die Lösung kann in Mund oder Nase gesprüht werden. Ein Sprühstoß (etwa 0,1 ml) entspricht einer Dosis von etwa 0,14 mg.

## Patentansprüche

1. Verbindungen ausgewählt aus der Gruppe
4-[4-(3-Chlor-4-methoxy-benzylamino)-9-(1-phenyl-ethyl)-6,7,8,9-tetrahydro-5*H*-1,3,9-triaza-fluoren-2-yl]-benzoesäure,
4-[4-(3,4-Methylendioxy-benzylamino)-5,6,7,8-tetrahydrobenzo[4,5]furo[2,3-*d*]pyrimidin-2-yl]-benzoesäure,
4-[4-(3-Chlor-4-methoxy-benzylamino)-5,6,7,8-tetrahydrobenzo[4,5]furo[2,3-*d*]pyrimidin-2-yl]-benzoesäure,
4-(4-Benzylamino-5,6,7,8-tetrahydro-benzo[4,5]furo[2,3-*d*]pyrimidin-2-yl)-benzoesäure,
4-[4-(3,4-Dichlor-benzylamino)-5,6,7,8-tetrahydrobenzo[4,5]furo[2,3-*d*]pyrimidin-2-yl]-benzoesäure,
4-{4-[(Pyridin-3-ylmethyl)-amino]-5,6,7,8-tetrahydro-benzo[4,5]-furo[2,3-*d*]pyrimidin-2-yl]-benzoesäure,
4-[4-(3-Chlor-4-methoxy-benzylamino)-5,6,7,8-tetrahydrobenzo[4,5]furo[2,3-*d*]pyrimidin-2-yl]-buttersäure,
1-[4-(3-Chlor-4-methoxy-benzylamino)-5,6,7,8-tetrahydrobenzo[4,5]-furo[2,3-*d*]pyrimidin-2-ylmethyl]-piperidin-4-carbonsäure,
1-[4-(3-Chlor-4-methoxy-benzylamino)-5,6,7,8-tetrahydrobenzo[4,5]-furo[2,3-*d*]pyrimidin-2-ylmethyl]-4-methylsulfonylpiperazin,
4-[4-(3-Chlor-4-methoxy-benzylamino)-9*H*-1,3,9-triaza-fluoren-2-yl]-buttersäure,
4-[4-(3-Chlor-4-methoxy-benzylamino)-9*H*-1,3,9-triaza-fluoren-2-yl]-benzoesäure,
4-[4-(3,4-Methylendioxy-benzylamino)-9-ethyl-9*H*-1,3,9-triazafluoren-2-yl]-benzoesäure,
4-[4-(3-Chlor-4-methoxy-benzylamino)-9-methyl-9*H*-1,3,9-triazafluoren-2-yl]-benzoesäure,
4-[4-(3,4-Methylendioxy-benzylamino)-9-methyl-9*H*-1,3,9-triaza-fluoren-2-yl]-benzoesäure,
5-[4-(3-Chlor-4-methoxy-benzylamino)-9-methyl-9*H*-1,3,9-triaza-fluoren-2-yl]-pentansäure,
4-[4-(3-Chlor-4-methoxy-benzylamino)-9-benzyl-9*H*-1,3,9-triaza-fluoren-2-yl]-benzoesäure,
4-[4-(3-Chlor-4-methoxy-benzylamino)-9-isopropyl-9*H*-1,3,9-triaza-fluoren-2-yl]-benzoesäure,
sowie ihre pharmazeutisch verwendbaren Derivate, Solvate und Stereoisomere.

2. Arzneimittel, enthaltend mindestens eine Verbindung nach Anspruch 1 und/oder ihre pharmazeutisch verwendbaren Derivate, Solvate und Stereoisomere, einschließlich deren Mischungen in allen Verhältnissen, sowie gegebenenfalls Träger- und/oder Hilfsstoffe.

3. Verwendung von Verbindungen nach Anspruch 1 und/oder ihre physiologisch unbedenklichen Salze und Solvate zur Herstellung eines Arzneimittels zur Behandlung von Angina, Bluthochdruck, pulmonarem Hochdruck, congestivem Herzversagen, Herzinfarkt, chronischer obstruktiver pulmonaler Krankheit (COPD), Cor pulmonale, Rechtsherzinsuffizienz, Atherosklerose, Bedingungen verminderter Durchgängigkeit der Herzgefäße, peripheren vaskulären Krankheiten, Schlaganfall, Bronchitis, allergischem Asthma, chronischem Asthma, allergischer Rhinitis, Glaucom, Irritable Bowel Syndrome, Tumoren, Niereninsuffizienz, Leberzirrhose, zur Behandlung weiblicher Sexualstörungen, Entzündungen, Osteoporose, zur Behandlung von maligner Hypertonie, Phäochromazytom, peripherer Gefäß(-verschluß)-erkrankungen, Gefäßerkrankungen, Thrombozytopenie, Ulcus pepticum, Darmbewegungsstörungen, perkutaner transluminaler Koronarangioplastie, Karotis Angioplastie, postoperativer Stenose des Koronarbypasses, Vorwehen und benigner Prostata-Hyperplasie.

4. Verwendung von Verbindungen nach Anspruch 1 und/oder ihre physiologisch unbedenklichen Salze und Solvate zur Herstellung eines Arzneimittels zur Behandlung von Krankheiten des Herz-Kreislaufsystems und zur Behandlung und/oder Therapie von Potenzstörungen.

## Claims

1. Compounds selected from the group
4-[4-(3-chloro-4-methoxybenzylamino)-9-(1-phenylethyl)-6,7,8,9-tetrahydro-5*H*-1,3,9-triazafluoren-2-yl]benzoic acid,
4-[4-(3,4-methylenedioxybenzylamino)-5,6,7,8-tetrahydrobenzo-[4,5]furo[2,3-*d*]pyrimidin-2-yl]benzoic acid,
4-[4-(3-chloro-4-methoxybenzylamino)-5,6,7,8-tetrahydrobenzo-[4,5]furo[2,3-*d*]pyrimidin-2-yl]benzoic acid,
4-(4-benzylamino-5,6,7,8-tetrahydrobenzo[4,5]furo[2,3-*d*]pyrimidin-2-yl)benzoic acid,
4-[4-(3,4-dichlorobenzylamino)-5,6,7,8-tetrahydrobenzo[4,5]-furo[2,3-*d*]pyrimidin-2-yl]benzoic acid,
4-{4-[(pyridin-3-ylmethyl)amino]-5,6,7,8-tetrahydrobenzo[4,5]-furo[2,3-*d*]pyrimidin-2-yl}benzoic acid,
4-[4-(3-chloro-4-methoxybenzylamino)-5,6,7,8-tetrahydrobenzo-[4,5]furo[2,3-*d*]pyrimidin-2-yl]butyric acid,
1-[4-(3-chloro-4-methoxybenzylamino)-5,6,7,8-tetrahydrobenzo-[4,5]furo[2,3-*d*]pyrimidin-2-ylmethyl]piperidine-4-carboxylic acid,
1-[4-(3-chloro-4-methoxybenzylamino)-5,6,7,8-tetrahydrobenzo-[4,5]furo[2,3-*d*]pyrimidin-2-ylmethyl]-4-methylsulfonylpiperazine,
4-[4-(3-chloro-4-methoxybenzylamino)-9*H*-1,3,9-triazafluoren-2-yl]butyric acid,
4-[4-(3-chloro-4-methoxybenzylamino)-9*H*-1,3,9-triazafluoren-2-yl]benzoic acid,
4-[4-(3,4-methylenedioxybenzylamino)-9-ethyl-9*H*-1,3,9-triazafluoren-2-yl]benzoic acid,
4-[4-(3-chloro-4-methoxybenzylamino)-9-methyl-9*H*-1,3,9-triazafluoren-2-yl]benzoic acid,
4-[4-(3,4-methylenedioxybenzylamino)-9-methyl-9*H*-1,3,9-triazafluoren-2-yl]benzoic acid,
5-[4-(3-chloro-4-methoxybenzylamino)-9-methyl-9*H*-1,3,9-triazafluoren-2-yl]pentanoic acid,
4-[4-(3-chloro-4-methoxybenzylamino)-9-benzyl-9*H*-1,3,9-triazafluoren-2-yl]benzoic acid,
4-[4-(3-chloro-4-methoxybenzylamino)-9-isopropyl-9*H*-1,3,9-triazafluoren-2-yl]benzoic acid,
and pharmaceutically usable derivatives, solvates and stereoisomers thereof.

2. Medicaments comprising at least one compound according to Claim 1 and/or pharmaceutically usable derivatives, solvates and stereoisomers thereof, including mixtures thereof in all ratios, and, if desired, excipients and/or adjuvants.

3. Use of compounds according to Claim 1 and/or physiologically acceptable salts and solvates thereof for the preparation of a medicament for the treatment of angina, high blood pressure, pulmonary hypertension, congestive heart failure, cardiac infarction, chronic obstructive pulmonary disease (COPD), cor pulmonale, dextrocardiac insufficiency, atherosclerosis, conditions of reduced patency of the cardiac vessels, peripheral vascular diseases, strokes, bronchitis, allergic asthma, chronic asthma, allergic rhinitis, glaucoma, irritable bowel syndrome, tumours, renal insufficiency, liver cirrhosis, for the treatment of female sexual disorders, inflammation, osteoporosis, for the treatment of malignant hypertonia, phaeochromacytoma, peripheral vascular (occlusion) diseases, vascular diseases, thrombocytopenia, peptic ulcer, peristaltic motion disorders, percutaneous transluminal coronary angioplasty, carotid angioplasty, postoperative stenosis of the coronary bypass, premonitory pains and benign prostatic hyperplasia.

4. Use of compounds according to Claim 1 and/or physiologically acceptable salts and solvates thereof for the preparation of a medicament for the treatment of diseases of the cardiovascular system and for the treatment and/or therapy of impotence.

## Revendications

1. Composés sélectionnés parmi le groupe
l'acide 4-[4-(3-chloro-4-méthoxybenzylamino)-9-(1-phényléthyl)-6,7,8,9-tétrahydro-5*H*-1,3,9-triazafluorén-2-yl]benzoïque,
l'acide 4-[4-(3,4-méthylènedioxybenzylamino)-5,6,7,8-tétrahydrobenzo[4,5]furo[2,3-*d*]pyrimidin-2-yl]benzoïque,
l'acide 4-[4-(3-chloro-4-méthoxybenzylamino)-5,6,7,8-tétrahydrobenzo[4,5]furo[2,3-*d*]pyrimidin-2-yl]benzoïque,
l'acide 4-(4-benzylamino-5,6,7,8-tétrahydrobenzo[4,5]furo-[2,3-*d*]pyrimidin-2-yl)benzoïque,
l'acide 4-[4-(3,4-dichlorobenzylamino)-5,6,7,8-tétrahydrobenzo-[4,5]furo[2,3-*d*]pyrimidin-2-yl]benzoïque,
l'acide 4-{4-[(pyridin-3-ylméthyl)amino]-5,6,7,8-tétrahydrobenzo-[4,5]furo[2,3-*d*]pyrimidin-2-yl}benzoïque,
l'acide 4-[4-(3-chloro-4-méthoxybenzylamino)-5,6,7,8-tétrahydrobenzo[4,5]furo[2,3-*d*]pyrimidin-2-yl]butyrique,
l'acide 1-[4-(3-chloro-4-méthoxybenzylamino)-5,6,7,8-tétrahydrobenzo[4,5]furo[2,3-*d*]pyrimidin-2-ylméthyl]pipéridine-4-carboxylique,
la 1-[4-(3-chloro-4-méthoxybenzylamino)-5,6,7,8-tétrahydrobenzo[4,5]furo[2,3-*d*]pyrimidin-2-ylméthyl]-4-méthylsulfonylpipérazine,
l'acide 4-[4-(3-chloro-4-méthoxybenzylamino)-9*H*-1,3,9-triazafluorén-2-yl]butyrique,
l'acide 4-[4-(3-chloro-4-méthoxybenzylamino)-9*H*-1,3,9-triazafluorén-2-yl]benzoïque,
l'acide 4-[4-(3,4-méthylènedioxybenzylamino)-9-éthyl-9*H*-1,3,9-triazafluorén-2-yl]benzoïque,
l'acide 4-[4-(3-chloro-4-méthoxybenzylamino)-9-méthyl-9*H-*1,3,9-triazafluorén-2-yl]benzoïque,
l'acide 4-[4-(3,4-méthylènedioxybenzylamino)-9-méthyl-9*H-*1,3,9-triazafluorén-2-yl]benzoïque,
l'acide 5-[4-(3-chloro-4-méthoxybenzylamino)-9-méthyl-9*H-*1,3,9-triazafluorén-2-yl]pentanoïque,
l'acide 4-[4-(3-chloro-4-méthoxybenzylamino)-9-benzyl-9*H-*1,3,9-triazafluorén-2-yl]benzoïque,
l'acide 4-[4-(3-chloro-4-méthoxybenzylamino)-9-isopropyl-9*H-*1,3,9-triazafluorén-2-yl]benzoïque,
et des dérivés, solvats et stéréoisomères pharmaceutiquement utilisables de ceux-ci.

2. Médicaments comprenant au moins un composé selon la revendication 1 et/ou des dérivés, solvats et stéréoisomères pharmaceutiquement utilisables de ceux-ci, y compris des mélanges de ceux-ci en toutes proportions, et, si désiré, des excipients et/ou adjuvants.

3. Utilisation de composés selon la revendication 1 et/ou de sels et de solvats physiologiquement acceptables de ceux-ci pour la préparation d'un médicament destiné au traitement de l'angine, de l'hypertension artérielle, de l'hypertension pulmonaire, de l'insuffisance cardiaque congestive, de l'infarctus du myocarde, de la bronchopneumopathie chronique obstructive (COLD), du coeur pulmonaire, de l'insuffisance cardiaque droite, de l'athérosclérose, de conditions de patence réduite des vaisseaux cardiaques, de maladies vasculaires périphériques, d'accidents cérébrovasculaires, de la bronchite, de l'asthme allergique, de l'asthme chronique, de la rhinite allergique, du glaucome, du syndrome du colon irritable, de tumeurs, de l'insuffisance rénale, de la cirrhose du foie, au traitement de troubles sexuels féminins, d'inflammations, de l'ostéoporose, au traitement de l'hypertonie maligne, du phéochromocytome, de maladies vasculaires périphériques (occlusion), de maladies vasculaires, de la thrombocytopénie, de l'ulcère gastroduodénal, de troubles du péristaltisme, de l'angioplastie coronaire transluminale percutanée, de l'angioplastie carotidienne, de la sténose postopératoire du pontage coronarien, des douleurs prémonitoires et de l'hyperplasie bénigne de la prostate.

4. Utilisation de composés selon la revendication 1 et/ou de sels et de solvats physiologiquement acceptables de ceux-ci pour la préparation d'un médicament destiné au traitement de maladies du système cardiovasculaire et pour le traitement et/ou la thérapie de l'impuissance.
